# EUROPEAN PATENT APPLICATION

(11) **EP 3 673 907 A1**
(43) Date of publication of application: **01.07.2020**
(21) Application number: 18848560.1
(22) Date of filing: 22.08.2018
(51) Int. Cl.: A61K 31/4709, A61K 31/395, A61K 31/4184, A61K 31/445, A61K 31/496, A61K 31/497, A61K 31/519, A61K 31/52, A61K 31/5383, A61K 31/56, A61K 31/573, A61K 31/704, A61K 31/706, A61K 31/7068, A61K 45/00, A61P 35/00, A61P 35/02, A61P 35/04, A61P 43/00

(54) **PHARMACEUTICAL FOR CANCER TREATMENT INCLUDING AX1 INHIBITOR AS AN EFFECTIVE COMPONENT**

(30) Priority: 23.08.2017 JP 2017159839; 23.08.2017 JP 2017159837; 23.08.2017 JP 2017159841
(71) Applicant: ONO Pharmaceutical Co., Ltd., Osaka-shi, Osaka 541-8526 (JP)
(72) Inventor: YASUHIRO, Tomoko, Mishima-gun Osaka 618-8585 (JP); TANAKA, Kohei, Mishima-gun Osaka 618-8585 (JP)
(74) Representative: Grünecker Patent- und Rechtsanwälte PartG mbB
(86) International application number: PCT/JP2018/031047
(87) International publication number: WO 2019/039525

(57) **Abstract**

The present invention addresses the problem of identifying an effective method for curing cancer and providing a pharmaceutical product for cancer treatment. Provided is a pharmaceutical for cancer treatment, characterized by being administered as the combination of an Axl inhibitor and an anti-cancer agent (e.g., cytotoxic anti-cancer agent, molecularly targeted drug, and anti-CTLA-4 antibody), the Axl inhibitor including a compound represented by general formula (I) (in the formula, all symbols are as defined in the description), a salt thereof, a solvate thereof, an N-oxide substance thereof, or a prodrug of any of them. This combination exhibits a strong anti-tumor effect and is therefore useful for treatment of hematological cancer and/or solid cancer.

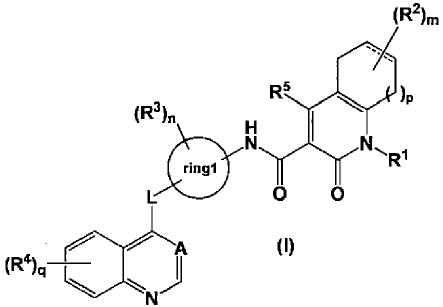

## Description

### [Technical Field]

The present invention, in one embodiment, relates to an agent for cancer treatment, containing an Axl inhibitor as an active ingredient, which is administered in combination with an anticancer drug, wherein the Axl inhibitor is a compound represented by the general formula (I): 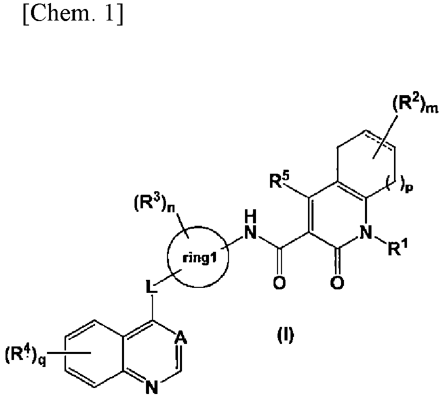 (wherein all of the symbols have the same meanings as given below), a salt thereof, a solvate thereof, an N-oxide thereof, or a prodrug thereof.

### [Background Art]

Blood cancers such as leukemia, lymphoma, and myeloma are in a field in which the pathologic conditions are revealed one after another in the molecular and genetic level, and diagnosis method and treatment method are rapidly advancing. In addition to conventional standard chemotherapy, molecular targeted therapy, hematopoietic stem cell transplantation, antitumor immunization therapy, and adjuvant therapy are newly reported every year, thus expanding treatment options (see Non-Patent Literature 1).

Axl (also known as: UFO, ARK, Tyro7) is a receptor tyrosine kinase belonging to a TAM family (Axl, Mer and Tyro3) cloned from tumor cells. Gas6 (growth-arrest-specific protein 6) cloned as a gene specifically expressed at the time of cell proliferation arrest is known as a ligand for Axl. Axl activated by binding of Gas6 transfers a signal via phosphorylation. Since the signal activates an Erk1/2 pathway or a PI3K/Akt pathway, the activation of Axl is known to be involved in pathologic conditions of cancers, immune system diseases, circulatory system diseases, and the like (see, Non-Patent Literature 2).

In particular, the relation between Axl and various types of cancers is well known. For example, it is known that the expression of Axl is involved in metastasis and prognosis of breast cancer (see, Non-Patent Literature 3), and that Axl is involved in the pathologic conditions of acute myeloid leukemia (AML) (see Non-Patent Literature 4). Furthermore, it is reported that a TAM family including Axl is expressed in immunocytes such as a dendritic cell or a macrophage, and suppressively regulates antitumor immunization (see, Non-Patent Literature 5). Therefore, it is considered that compounds which inhibit the activation of Axl are useful for treatment of various type of cancers, immune system diseases, and circulatory system diseases.

Patent Literature 1 discloses that a compound represented by the general formula (I) has Axl inhibitory action, and is useful as an agent for cancer treatment (see, Patent Literature 1).

Patent Literature 2 discloses that combination of a compound represented by the general formula (I) and an immune checkpoint inhibitor is useful for cancer treatment (see Patent Literature 2).

### [Citation List]

### [Patent Literatures]

[Patent Literature 1] International Publication WO2015/012298
[Patent Literature 2] International Publication WO2017/146236

### [Non-Patent Literature]

[Non-Patent Literature 1] Guideline for tumors of hematopoietic and lymphoid tissues of the Japanese Society of Hematology, 2013
[Non-Patent Literature 2] Clinical Science, Vol. 122, pp. 361-368, 2012
[Non-Patent Literature 3] Proceedings of the national academy of sciences of the United States of America, Vol. 107, No.3, pp. 1124-1129, 2010
[Non-Patent Literature 4] Blood, Vol. 121, pp.2064-2073, 2013
[Non-Patent Literature 5] Nature Reviews Cancer, Vol. 14, pp. 769-785, 2014

### [Summary of Invention]

### [Technical Problem]

A problem to be solved by the present invention is to find combination of agents useful for cancer treatment and to provide the combination as a pharmaceutical preparation.

### [Solution to problem]

In order to solve the above-mentioned problem, the inventors of the present invention have keenly studied. As a result, the inventors have found that the above-mentioned problem can be solved by a combination of an Axl inhibitor and an anticancer drug (hereinafter, also referred to as a "combination of the present invention"), wherein the Axl inhibitor is a compound represented by the following general formula (I), a salt thereof, a solvate thereof, an N-oxide thereof, or a prodrug thereof.

In other words, the present invention relates to:
[1] an agent for cancer treatment, containing an Axl inhibitor as an active ingredient, which is administered in combination with an anticancer drug,
[2] an agent for cancer treatment, containing an anticancer drug as an active ingredient, which is administered in combination with an Axl inhibitor,
[3] a method for treating cancer, which includes administering an effective amount of an Axl inhibitor in combination with an anticancer drug to mammalian (preferably, a human patient) in need of treatment of cancer,
[4] an Axl inhibitor to be used for cancer treatment in combination with an anticancer drug, and
[5] an Axl inhibitor for the manufacture of an agent for cancer treatment, which is administered in combination with an anticancer drug.

### [Advantageous Effects of Invention]

A combination of the present invention is useful for cancer treatment.

### [Brief Description of Drawings]

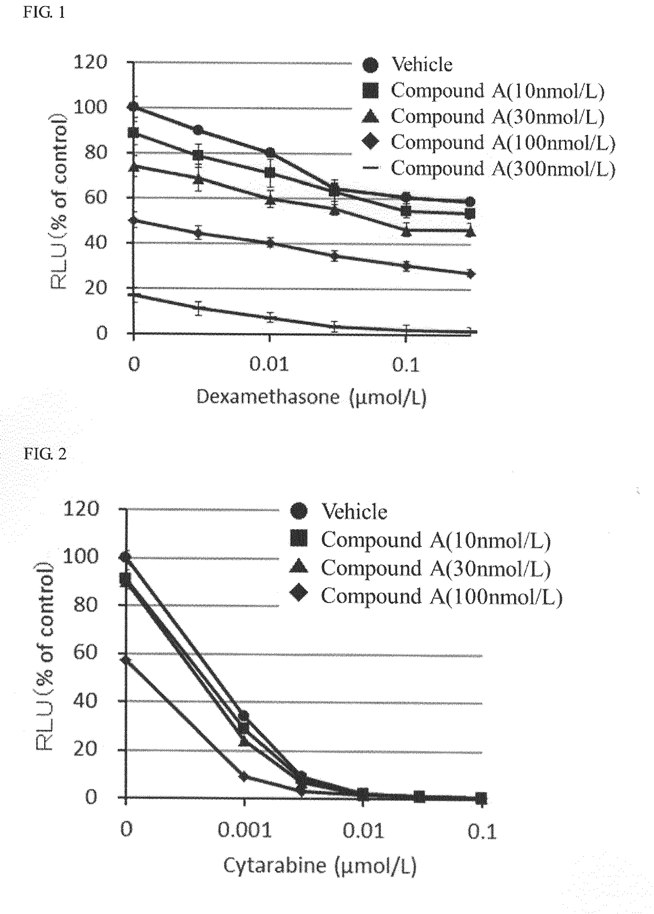

FIG. 1 shows a proliferation-suppressing action on a human acute lymphatic leukemia cell line CCRF-HSB-2 by a single use or combined use of N-{5-[(6,7-dimethoxy-4-quinolinyl)oxy]-2-pyridinyl}-2,5-dioxo-1-phenyl-1,2,5,6,7,8-hexahydro-3-quinolinecarboxamide (a compound A in the drawing) and dexamethasone. The ordinate shows a percentage (%) of relative luminescence unit (RLU) of each compound-treated group with respect to RLU in the vehicle group, and abscissa shows the concentration of dexamethasone. Values of each group shows a mean value ± standard error.
FIG. 2 shows a proliferation-suppressing action on a human acute lymphatic leukemia cell line CCRF-HSB-2 by a single use or combined use of N-{5-[(6,7-dimethoxy-4-quinolinyl)oxy]-2-pyridinyl}-2,5-dioxo-1-phenyl-1,2,5,6,7,8-hexahydro-3-quinolinecarboxamide (a compound A in the drawing) and Cytarabine. The ordinate shows a percentage (%) of RLU of each compound-treated group with respect to RLU in the vehicle group, and abscissa shows the concentration of Cytarabine. Values of each group shows a mean value ± standard error.

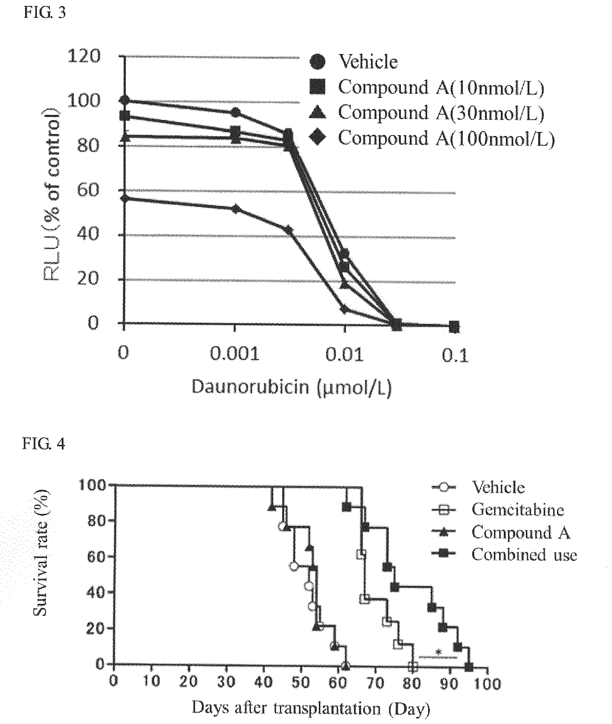

FIG. 3 shows a proliferation-suppressing action on a human acute lymphatic leukemia cell line CCRF-HSB-2 by a single use or combined use of N-{5-[(6,7-dimethoxy-4-quinolinyl)oxy]-2-pyridinyl}-2,5-dioxo-1-phenyl-1,2,5,6,7,8-hexahydro-3-quinolinecarboxamide (a compound A in the drawing) and daunorubicin. The ordinate shows a percentage (%) of RLU of each compound-treated group with respect to RLU in the vehicle group, and abscissa shows the concentration of daunorubicin. Values of each group shows a mean value ± standard error.
FIG. 4 shows an antitumor action on a mouse pancreatic cancer cell line Pan02 orthotopic implantation model by N-{5-[(6,7-dimethoxy-4-quinolinyl)oxy]-2-pyridinyl}-2,5-dioxo-1-phenyl-1,2,5,6,7,8-hexahydro-3-quinolinecarboxamide (compound A in the drawing) and gemcitabine. The ordinate shows a survival rate (%), and the abscissa shows days after transplantation (on day 0, each administration is started).

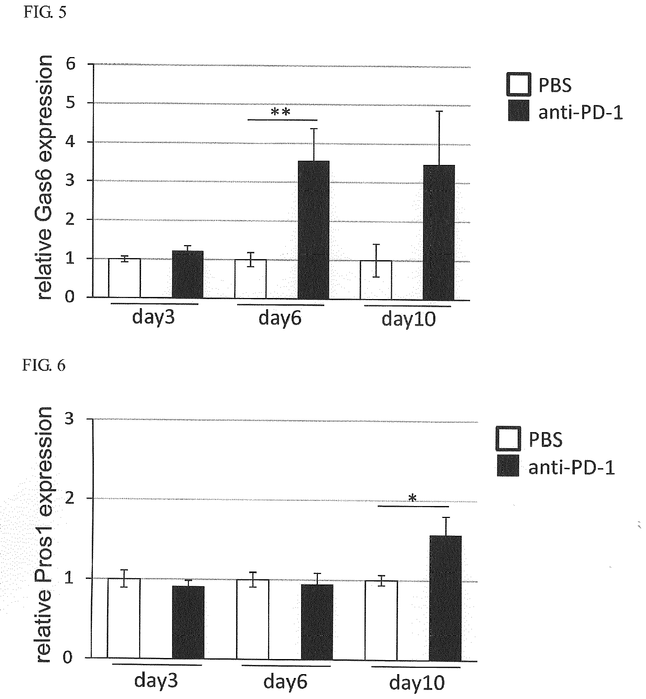

FIG. 5 shows a change amount of Axl and Mer ligand (Gas6) in tumor after administration of an anti-PD-1 antibody in a mouse large-intestine cancer cell line MC38 subcutaneous cancer-bearing model. The ordinate shows a relative expression amount of each ligand, and the abscissa shows days after administration of the anti-PD-1 antibody.
FIG. 6 shows a change amount of Axl and Mer ligand (Pros1) in tumor after administration of an anti-PD-1 antibody in a mouse large-intestine cancer cell line MC38 subcutaneous cancer-bearing model. The ordinate shows an expression amount of each ligand, and the abscissa shows days after administration of the anti-PD-1 antibody.

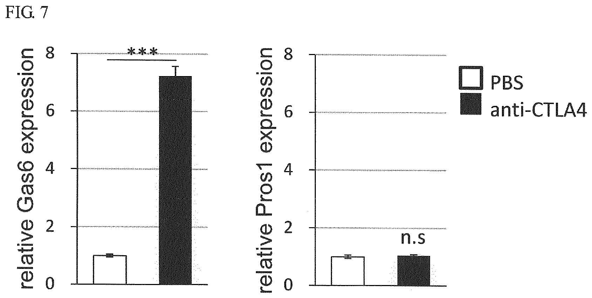

FIG. 7 shows a change amount of Axl and Mer ligand (Gas6 (left) and Pros1 (right)) in the immunocyte by an anti-CTLA-4 antibody using CTLA-4 Blockade Bioassay. The ordinate shows the relative expression amount of each ligand.

### [Description of Embodiments]

### (1) Axl inhibitor

In one embodiment, an Axl inhibitor to be used for the combination of the present invention is a compound represented by the general formula (I) described in WO2015/012298: 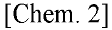 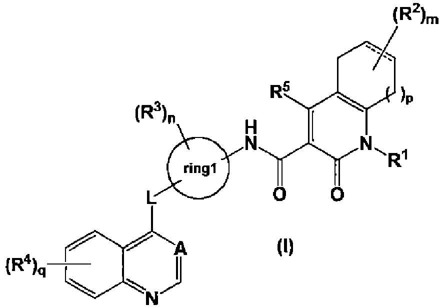
[wherein in the formula, R¹ represents (1) a C1-8 alkyl group optionally substituted with one to five R¹¹, (2) a C3-7 carbocyclic ring optionally substituted with one to five R¹²; or (3) a 4-to 7-membered heterocycle optionally substituted with one to five R¹³;
herein when the C1-8 alkyl group represented by R¹ is a branched alkyl group, C1-3 alkyl groups branched from the same carbon atom, together with a carbon atom bonded thereto, may form a saturated C3-7 carbocyclic ring;
R² represents (1) a C1-4 alkyl group, (2) a halogen atom, (3) a C1-4 haloalkyl group, (4) an oxo group, (5) an -OR²¹ group, or (6) a =NR²² group;
R³ represents (1) a C1-4 alkyl group, (2) a halogen atom, or (3) a C1-4 haloalkyl group;
R⁴ represents (1) a C1-4 alkoxy group, (2) a C1-4 haloalkyl group, or (3) an -OR⁴¹ group; (4) a C1-4 alkyl group, (5) a C2-4 alkenyloxy group, or (6) a C2-4 alkynyloxy group;
R⁵ represents (1) a hydrogen atom, (2) a C1-4 alkyl group, (3) a halogen atom, (4) a C1-4 haloalkyl group, or (5) an -OR²¹ group;
R¹¹ represents (1) an -OR¹⁰¹ group, (2) an SO₂R¹⁰² group, (3) an NR¹⁰³R¹⁰⁴ group, or (4) a C3-7 carbocyclic ring optionally substituted with one to three halogen atoms;
R¹² represents (1) a C1-8 alkyl group optionally substituted with a hydroxyl group, or (2) a halogen atom;
R¹³ represents (1) a C1-8 alkyl group optionally substituted with a hydroxyl group, or (2) a halogen atom;
R²¹ represents (1) a hydrogen atom, or (2) a C1-4 alkyl group;
R²² represents (1) a hydroxyl group, or (2) a C1-4 alkoxy group;
R⁴¹ represents (1) a hydrogen atom, (2) a C1-8 alkyl group substituted with one or two substituents selected from the group consisting of (a) a 5- to 7-cyclic group optionally substituted with one or two substituents selected from the group consisting of (i) a C1-4 alkyl group, (ii) a C1-4 haloalkyl group, and (iii) a halogen atom, (b) NR⁴⁰¹R⁴⁰², (c) a hydroxyl group, and (d) a SO₂R⁴⁰³ group,
(3) a C2-8 alkenyl group substituted with one or two substituents selected from the group consisting of (a) a 5- to 7-cyclic group optionally substituted with one or two substituents selected from the group consisting of (i) a C1-4 alkyl group, (ii) a C1-4 haloalkyl group, and (iii) a halogen atom, (b) NR⁴⁰¹R⁴⁰², (c) a hydroxyl group, and (d) a SO₂R⁴⁰³ group, or
(4) a C2-8 alkynyl group substituted with one or two substituents selected from the group consisting of (a) a 5- to 7-cyclic group optionally substituted with one or two substituents selected from the group consisting of (i) a C1-4 alkyl group, (ii) a C1-4 haloalkyl group, and (iii) a halogen atom, (b) NR⁴⁰¹R⁴⁰², (c) a hydroxyl group, and (d) a SO₂R⁴⁰³ group;
R¹⁰¹ represents (1) a hydrogen atom, or (2) a C1-4 alkyl group;
R¹⁰² represents (1) a hydrogen atom, or (2) a C1-4 alkyl group;
R¹⁰³ and R¹⁰⁴ each independently represent (1) a hydrogen atom, or (2) a C1-4 alkyl group;
R⁴⁰¹ and R⁴⁰² each independently represent (1) a hydrogen atom, or (2) a C1-4 alkyl group;
R⁴⁰³ represents (1) a hydrogen atom, or (2) a C1-4 alkyl group;
A represents (1) CH, or (2) a nitrogen atom;
L represents (1) -O-, (2) -NH-, (3) -C(O)-, (4) -CR⁶R⁷-, (5) -S-, (6) -S(O)-, or (7) -S(O)₂-;
R⁶ and R⁷ each independently represent (1) a hydrogen atom, (2) a halogen atom, (3) a C1-4 alkyl group, (4) a hydroxyl group, or (5) NH₂;
ring1 represents a 5- to 7-membered cyclic group; 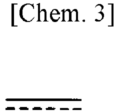
represents a single bond or a double bond;
m represents an integer of 0 to 5;
n represents an integer of 0 to 5;
p represents an integer of 0 to 2;
q represents an integer of 0 to 4;
when m is 2 or more, a plurality of R²'s may be the same as or different from each other, and herein when two R²'s represent a C1-3 alkyl group and are on the same carbon atom, the R²'s, together with a carbon atom bonded thereto, may form a C3-7 saturated carbocyclic ring; when n is 2 or more, a plurality of R³'s may be the same as or different from each other; and when q is 2 or more, a plurality of R⁴'s may be the same as or different from each other], a salt thereof, a solvate thereof, an N-oxide thereof, or a prodrug thereof.

Further embodiments of the Axl inhibitor include a compound described in International Publication WO2007/030680, WO2007/057399, WO2007/070872, WO2008/045978, WO2008/080134, WO2008/083356, WO2008/128072, WO2008/083353, WO2008/083354, WO2008/083367, WO2008/083357, WO2009/007390, WO2009/024825, WO2009/047514, WO2009/053737, WO2009/054864, WO2009/127417, WO2010/005876, WO2010/005879, WO2010/090764, WO2010/128659, WO2012/028332, WO2012/135800, WO2013/074633, WO2013/115280, WO2013/162061, WO2014/091265, WO2016/006706, WO2016/097918, WO2016/183071, WO2017/028797, WO2017/172596, or WO2018/071343.

In the present invention, a halogen atom refers to fluorine, chlorine, bromine, and iodine.

In the present invention, the C1-8 alkyl group includes a linear or branched C1-8 alkyl group. Examples thereof include a methyl group, an ethyl group, a propyl group, a butyl group, a pentyl group, a hexyl group, a heptyl group, an octyl group, an isopropyl group, an isobutyl group, a sec-butyl group, a tert-butyl group, and isomers thereof.

In the present invention, the C1-4 alkyl group includes a linear or branched C1-4 alkyl group. Examples thereof include a methyl group, an ethyl group, a propyl group, a butyl group, an isopropyl group, an isobutyl group, a sec-butyl group, and a tert-butyl group.

In the present invention, the C1-3 alkyl group includes a linear or branched C1-3 alkyl group. Examples thereof include a methyl group, an ethyl group, a propyl group, and an isopropyl group.

In the present invention, the C1-4 haloalkyl group refers to, for example, a fluoromethyl group, a chloromethyl group, a bromomethyl group, an iodomethyl group, a difluoromethyl group, a trifluoromethyl group, a 1-fluoroethyl group, a 2-fluoroethyl group, a 2-chloroethyl group, a pentafluoroethyl group, a 1-fluoropropyl group, a 2-chloropropyl group, a 3-fluoropropyl group, a 3-chloropropyl group, a 4,4,4-trifluorobutyl group, and a 4-bromobutyl group.

In the present invention, the C2-8 alkenyl group refers to, for example, a vinyl group, a propenyl group, a butenyl group, a pentenyl group, a hexenyl group, a heptenyl group, and an octenyl group, and isomers thereof, and the like.

In the present invention, the C2-8 alkynyl group refers to, for example, an ethynyl group, a propynyl group, a butynyl group, a pentynyl group, a hexynyl group, a heptynyl group, an octynyl group, and isomers thereof.

In the present invention, examples of the C1-4 alkoxy group include a methoxy group, an ethoxy group, a propoxy group, an isopropoxy group, a butoxy group, an isobutoxy group, a sec-butoxy group, or a tert-butoxy group.

In the present invention, the C2-4 alkenyloxy group refers to, for example, vinyloxy, propenyloxy, butenyloxy, and isomers thereof, and the like.

In the present invention, the C2-4 alkynyloxy group refers to, for example, ethynyloxy, propynyloxy, butynyloxy, and isomers thereof, and the like.

In the present invention, the C3-7 carbocyclic ring refers to a C3-7 monocyclic carbocyclic ring, and the carbocyclic ring which may be partially or completely saturated, and examples thereof include cyclopropane, cyclobutane, cyclopentane, cyclohexane, cycloheptane, cyclobutene, cyclopentene, cyclohexene, cycloheptene, cyclobutadiene, cyclopentadiene, cyclohexadiene, cycloheptadiene, or benzene ring.

In the present invention, the C5-7 carbocyclic ring refers to a C5-7 monocyclic carbocyclic ring, and the carbocyclic ring which may be partially or completely saturated, and examples thereof include cyclopentane, cyclohexane, cycloheptane, cyclopentene, cyclohexene, cycloheptene, cyclopentadiene, cyclohexadiene, cycloheptadiene, or benzene ring.

In the present invention, examples of the saturated C3-7 carbocyclic ring include cyclopropane, cyclobutane, cyclopentane, cyclohexane, and cycloheptane.

In the present invention, the 4- to 7-membered heterocycle refers to 4- to 7-membered monocyclic heterocycle, which includes one to five heteroatoms selected from an oxygen atom, a nitrogen atom and a sulfur atom, and a part or all of which is saturated. Examples thereof include azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxetane, dihydrofuran, tetrahydrofuran, dihydropyran, tetrahydropyran, dihydrooxepine, tetrahydrooxepine, perhydrooxepine, thietane, dihydrothiophene, tetrahydrothiophene, dihydrothiopyran, tetrahydrothiopyran, dihydrothiepin, tetrahydrothiepin, perhydrothiepin, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepin, tetrahydrothiadiazepin, perhydrothiadiazepin, morpholine, thiomorpholine, oxathiane, dioxolane, dioxane, dithiolane, dithiane, pyrrole, imidazole, triazole, tetrazole, pyrazole, pyridine, pyrazine, pyrimidine, pyridazine, azepine, diazepine, furan, pyran, oxepin, thiophene, thiopyran, thiepin, oxazole, isoxazole, thiazole, isothiazole, furazan, oxadiazole, oxazine, oxadiazine, oxazepine, oxadiazepine, thiadiazole, thiazine, thiadiazine, thiazepine, or thiadiazepin ring.

In the present invention, the 5- to 7-membered cyclic group refers to a C5-7 carbocyclic ring and a 5- to 7-membered heterocycle. Herein, the C5-7 carbocyclic ring refers to the same meaning mentioned above, and the 5- to 7-membered heterocycle includes 5- to 7-membered unsaturated heterocycle and 5- to 7-membered saturated heterocycle. Examples of the 5- to 7-membered heterocycle include pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrofuran, tetrahydrofuran, dihydropyran, tetrahydropyran, dihydrooxepine, tetrahydrooxepine, perhydrooxepine, dihydrothiophene, tetrahydrothiophene, dihydrothiopyran, tetrahydrothiopyran, dihydrothiepin, tetrahydrothiepin, perhydrothiepin, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepin, tetrahydrothiadiazepin, perhydrothiadiazepin, morpholine, thiomorpholine, oxathiane, dioxolane, dioxane, dithiolane, dithiane, pyrrole, imidazole, triazole, tetrazole, pyrazole, pyridine, pyrazine, pyrimidine, pyridazine, azepine, diazepine, furan, pyran, oxepin, thiophene, thiopyran, thiepin, oxazole, isoxazole, thiazole, isothiazole, furazan, oxadiazole, oxazine, oxadiazine, oxazepine, oxadiazepine, thiadiazole, thiazine, thiadiazine, thiazepine, or thiadiazepin ring.

In the present invention, the 6-membered cyclic group refers to a C6 carbocyclic ring and a 6-membered heterocycle. Examples thereof include cyclohexane, cyclohexene, cyclohexadiene, benzene, pyridine, pyrazine, pyrimidine, pyridazine, pyran, thiopyran, oxazine, oxadiazine, thiazine, thiadiazine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydropyran, tetrahydropyran, dihydrothiopyran, tetrahydrothiopyran, dihydrooxazine, tetrahydrooxazine, dihydrooxadiazine, tetrahydrooxadiazine, dihydrothiazine, tetrahydrothiazine, dihydrothiadiazine, tetrahydrothiadiazine, morpholine, thiomorpholine, oxathiane, dioxane, and dithiane ring.

In the present invention, "when the C1-8 alkyl group represented by R¹ is a branched alkyl group, C1-3 alkyl groups branched from the same carbon atom together may form a saturated C3-7 carbocyclic ring" means that in a partial structure of the following general formula (I): 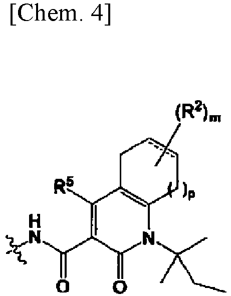 (wherein in the formula, all of the symbols have the same meanings as defined above), for example, when R¹ is a branched alkyl chain as represented in the above-mentioned formula, the alkyl chain branched from the same carbon atom, together with a carbon atom bonded thereto, forms a saturated carbocyclic ring, as shown in the following formula: 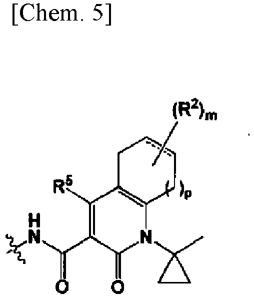 (wherein in the formula, all of the symbols have the same meanings as defined above).

In the present invention, "two R²'s represent a C1-3 alkyl group and are on the same carbon atom, the R²'s together with a carbon atom bonded thereto, may form a C3-7 saturated carbocyclic ring" means that in a partial structure of the following general formula (I): 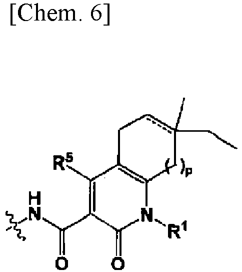 (wherein in the formula, all of the symbols have the same meanings as defined above), for example, when the R²'s are an alkyl group and on the same carbon, the R²'s together with a carbon atom bonded thereto form a saturated carbocyclic ring as shown in the following formula: 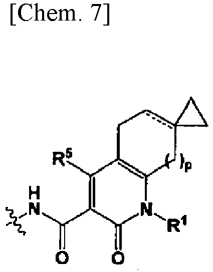 (wherein in the formula, all of the symbols have the same meanings as defined above).

In the present invention, "two R²⁻¹'s represent a C1-3 alkyl group and are on the same carbon atom, the R²⁻¹'s together with a carbon atom bonded thereto, may form a C3-7 saturated carbocyclic ring" has the same meaning as R² in the above-mentioned "two R²'s represent a C1-3 alkyl group, and are on the same carbon atom, the R²'s together with a carbon atom bonded thereto, may form a C3-7 saturated carbocyclic ring".

In the present invention, when m is one or more, one of the R²'s is preferably an oxo group.

In the present invention, A is preferably CH.

In the present invention, R⁴ is preferably a C1-4 alkoxy group or an -OR⁴¹ group.

In the present invention, L is preferably -O-, -NH-, or -C(O)-.

In the present invention, ring1 is preferably a 6-membered cyclic group, and more preferably benzene or pyridine.

The Axl inhibitor to be used for the combination of the present invention is preferably a compound represented by the general formula (I-1): 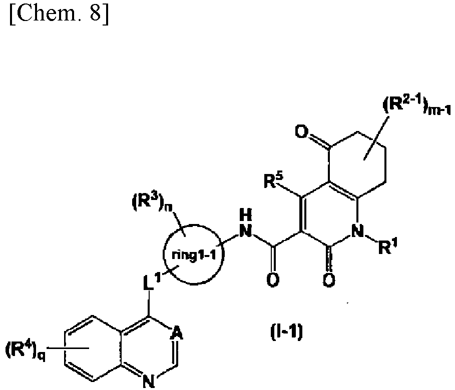
[wherein in the formula, R²⁻¹ represents (1) a C1-4 alkyl group, (2) a halogen atom, (3) a C1-4 haloalkyl group, (4) an -OR²¹ group, or (5) a =NR²² group; m-1 represents an integer of 0 to 4;
L¹ represents (1) -O-, (2) -NH-, or (3) -C(O)- ;
ring1-1 represents benzene or pyridine;
when m-1 is 2 or more, a plurality of R²⁻¹'s may be the same as or different from each other;
herein when the two R²⁻¹'s represent a C1-3 alkyl group and are on the same carbon atom, the R²⁻¹'s together with a carbon atom bonded thereto, may form a C3-7 saturated carbocyclic ring, and the other symbols have the same meanings as defined above], a salt thereof, a solvate thereof, an N-oxide thereof, or a prodrug thereof.

In the present invention, two binding arms in the ring1 and ring1-1 are preferably bonded to the para position.

In the present invention, in the general formula (1-1), A is preferably CH, R⁴ is preferably a C1-4 alkoxy group, or an -OR⁴¹ group.

The Axl inhibitor to be used for the combination of the present invention is more preferably a compound described in Example of WO2015/012298, and a pharmaceutically acceptable salt thereof or a hydrate thereof, and further more preferably (1) N-{5-[(6,7-dimethoxy-4-quinolinyl)oxy]-2-pyridinyl}-2,5-dioxo-1-phenyl-1,2,5,6,7,8-hexahydro-3-quinolinecarboxamide, (2) N-{5-[(6,7-dimethoxy-4-quinolinyl)oxy]-2-pyridinyl}-7,7-dimethyl-2,5-dioxo-1-phenyl-1,2,5,6,7,8-hexahydro-3-quinolinecarboxamide, (3) N-{5-[(6,7-dimethoxy-4-quinolinyl)oxy]-2-pyridinyl}-1-(2,2-dimethylpropyl)-2,5-dioxo-1,2,5,6,7,8-hexahydro-3-quinolinecarboxamide, (4) N-[5-({7-[3-(4-morpholinyl)propoxy]-4-quinolinyl}oxy)-2-pyridinyl]2,5-dioxo-1-phenyl-1,2,5,6,7,8-hexahydro-3-quinolinecarboxamide, (5) N-{4-[(6,7-dimethoxy-4-quinolinyl)oxy]-3-fluorophenyl}-2,5-dioxo-1-phenyl-1,2,5,6,7,8-hexahydro-3-quinolinecarboxamide, (6) N-{4-[(6,7-dimethoxy-4-quinolinyl)oxy]phenyl}-2,5-dioxo-1-phenyl-1,2,5,6,7,8-hexahydro-3-quinolinecarboxamide, (7) N-{5-[(6,7-dimethoxy-4-quinolinyl)oxy]-2-pyridinyl}-1-(4-fluorophenyl)-2,5-dioxo-1,2,5,6,7,8-hexahydro-3-quinolinecarboxamide, (8) N-{5-[(6,7-dimethoxy-4-quinolinyl)oxy]-2-pyridinyl}-1-(3-fluorophenyl)-2,5-dioxo-1,2,5,6,7,8-hexahydro-3-quinolinecarboxamide, (9) N-{5-[(6,7-dimethoxy-4-quinolinyl)oxy]-2-pyridinyl}-1-(2-fluorophenyl)-2,5-dioxo-1,2,5,6,7,8-hexahydro-3-quinolinecarboxamide, (10) N-{5-[(6,7-dimethoxy-4-quinazolinyl)oxy]-2-pyridinyl}-2,5-dioxo-1-phenyl-1,2,5,6,7,8-hexahydro-3-quinolinecarboxamide, (11) N-{5-[(6,7-dimethoxy-4-quinazolinyl)oxy]-2-pyridinyl}-1-(4-fluorophenyl)-2,5-dioxo-1,2,5,6,7,8-hexahydro-3-quinolinecarboxamide, (12) N-{5-[(6,7-dimethoxy-4-quinolinyl)oxy]-2-pyridinyl}-1-[(2S)-1-hydroxy-3-methyl-2-butanyl]-2,5-dioxo-1,2,5,6,7,8-hexahydro-3-quinolinecarboxamide, (13) N-{4-[(6,7-dimethoxy-4-quinolinyl)oxy]-3-fluorophenyl}-1-(3-fluorophenyl)-2,5-dioxo-1,2,5,6,7,8-hexahydro-3-quinolinecarboxamide, (14) N-{5-[(6,7-dimethoxy-4-quinolinyl)oxy]-2-pyridinyl}-6,6-dimethyl-2,5-dioxo-1-phenyl-1,2,5,6,7,8-hexahydro-3-quinolinecarboxamide, (15) N-[5-({6-methoxy-7-[3-(4-morpholinyl)propoxy]-4-quinolinyl}oxy)-2-pyridinyl]-2,5-dioxo-1-phenyl-1,2,5,6,7,8-hexahydro-3-quinolinecarboxamide, (16) N-(5-{[7-(3-hydroxy-3-methylbutoxy)-6-methoxy-4-quinolinyl]oxy}-2-pyridinyl)-2,5-dioxo-1-phenyl-1,2,5,6,7,8-hexahydro-3-quinolinecarboxamide, or (17) N-[5-({6-methoxy-7-[3-(1-pyrrolidinyl)propoxy]-4-quinolinyl}oxy)-2-pyridinyl]-2,5-dioxo-1-phenyl-1,2,5,6,7,8-hexahydro-3-quinolinecarboxamide, and a pharmaceutically acceptable salt thereof or a hydrate thereof.

Particularly preferred is N-{5-[(6,7-dimethoxy-4-quinolinyl)oxy]-2-pyridinyl}-2,5-dioxo-1-phenyl-1,2,5,6,7,8-hexahydro-3-quinolinecarboxamide (hereinafter, also abbreviated as a "compound A") represented by the following structural formula: 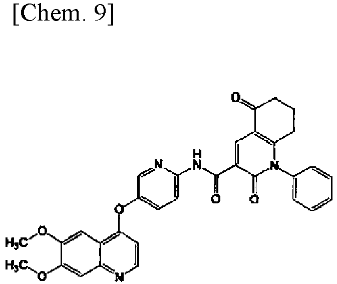 and a pharmaceutically acceptable salt thereof or a hydrate thereof.

In the present invention, unless specifically indicated, all of the isomers are included. For example, an alkyl group includes linear and branched chain groups. In addition, all of geometrical isomers of double bonds, rings, and fused rings (E-, Z-, cis-, trans-isomers), optical isomers by the presence of an asymmetric carbon atom (R-, S-isomer, α-, β-configurations, enantiomers, diastereomers), optical active isomers having optical rotation property (D, L, d, 1-isomers), polar isomers according to chromatographic separation (more polar isomer, less polar isomer), equilibrium compound, rotamers, mixtures thereof at any rate, and racemic mixtures are included in the present invention. Furthermore, the present invention also encompasses all isomers by tautomers.

Furthermore, the optical isomer of the present invention is not only limited to an optical isomer having purity of 100%, but also may include other optical isomers having purity of less than 50%.

In the present invention, unless otherwise noted, as apparent to a person skilled in the art, a symbol: 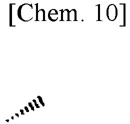 represents binding toward the back side of the plane of the paper (that is to say, the α-configuration), 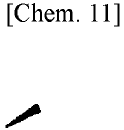 represents binding toward the front side of the plane of the paper (that is to say, the β-configuration), and 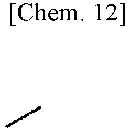 represents α-configuration, β-configuration or an arbitrary mixture thereof.

The compound represented by the general formula (I) is converted into a corresponding salt by the well-known method. A salt is preferably a pharmaceutically acceptable salt. Furthermore, the salt is preferably a water-soluble salt. Examples of a suitable salt include salts of an alkali metal (potassium, sodium, and the like), salts of an alkaline earth metal (calcium, magnesium, and the like), ammonium salts, salts of a pharmaceutically acceptable organic amine (tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arginine, N-methyl-D-glucamine, and the like), or acid addition salts (inorganic acid salts (hydrochloride, hydrobromide, hydroiodide, sulfate, phosphate, nitrate, and the like), organic acid salts (acetate, trifluoroacetate, lactate, tartrate, oxalate, fumarate, maleate, benzoate, citrate, methanesulfonate, ethanesulfonate, benzenesulfonate, toluenesulfonate, isethionate, glucuronate, gluconate, and the like).

The compound represented by the general formula (I) and a salt thereof can be also converted into a solvate. It is preferable that the solvate is low-toxic and water-soluble. Examples of a suitable solvate include solvates with water, or an alcoholic solvent (for example, ethanol). The solvate is preferably a hydrate.

The N-oxide of the compound represented by the general formula (I) refers to compounds represented by the general formula (I) in which a nitrogen atom is oxidized. Furthermore, the N-oxide of the compound represented by the general formula (I) may be salts of alkali (earth) metal salt, ammonium salt, organic amine salt, and acid addition salt mentioned above.

The prodrug of the compound represented by the general formula (I) refers to a compound which is converted into the compound represented by the general formula (I) by a reaction with an enzyme, stomach acid, and the like, in a living body. When the compound has a hydroxyl group, the prodrugs of the compound represented by the general formula (I) include: compounds in which the hydroxyl group is acylated, alkylated, phosphorylated, or borated (for example, the compounds to be used in combination of the present invention in which the hydroxyl group is acetylated, palmitoylated, propanoylated, pivaloylated, succinylated, fumarylated, alanylated, or dimethylaminomethylcarbonylated); and compounds represented by the general formula (I) in which the carboxyl group is esterified or amidated (for example, compounds represented by the general formula (I) in which the carboxyl group is made into ethyl ester, isopropyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methylamide, and the like). These compounds can be produced by well-known methods. Furthermore, the prodrug of the compound represented by the general formula (I) may be hydrate or non-hydrate. Furthermore, the prodrug of the compound represented by the general formula (I) may be a compound which is changed into the compound represented by the general formula (I) under the physiological condition, as described in "Development of Medicaments", vol. 7 "Molecular Design", pp. 163-198, published by Hirokawa Shoten in 1990. In addition, the compound represented by the general formula (I) may be labeled with an isotope thereof (for example, ²H, ³H, ¹¹C, ¹³C, ¹⁴C, ¹³N, ¹⁵N, ¹⁵O, ¹⁷O, ¹⁸O, ³⁵S, ¹⁸F, ³⁶Cl, ¹²³I, ¹²⁵I, and the like).

The compound represented by the general formula (I) can be produced according to the method described in WO2015/012298.

The Axl inhibitor to be used for the combination of the present invention is usually administered systemically or locally, by oral or parenteral administration. Examples of oral agents include liquid drugs for internal use (for example, elixirs, syrups, pharmaceutically acceptable water-based agents, suspensions, and emulsions), and solid drugs for internal use (for example, tablets (including sublingual tablets and orally disintegrating tablets), pills, capsules (including hard capsules, soft capsules, gelatin capsules, and microcapsules), powders, granules, and lozenges). Examples of parenteral agents include liquid drugs (for example, injections (subcutaneous injections, intravenous injections, intramuscular injections, intraperitoneal injections, and drip agents), eye drops (for example, aqueous eye drops (aqueous eye drops, aqueous eye drop suspensions, viscous eye drops, and solubilized eye drops, etc.), and nonaqueous eye drops (for example, nonaqueous eye drops and nonaqueous eye drop suspensions), and the like), agents for external use (for example, ointments (ophthalmic ointments, and the like)), and ear-drops, and the like. These formulations may be controlled release agents such as rapid release formulations, sustained release formulations, and the like. These formulations can be produced by well-known methods, for example, by the methods described in The Japanese Pharmacopoeia.

Liquid drugs for internal use as the oral agent can be produced by, for example, dissolving, suspending, or emulsifying an active ingredient in a generally used diluent (for example, purified water, ethanol, mixture liquid thereof, or the like). A liquid drug may include a wetting agent, a suspension agent, an emulsifying agent, a sweetening agent, a flavoring material, an aromatic substance, a preservative, a buffer agent, and the like.

Solid drugs for internal use as the oral agent are formulated by, for example, mixing the active ingredient with, for example, a vehicle (for example, lactose, mannitol, glucose, microcrystalline cellulose, and starch), a binder (for example, hydroxypropyl cellulose, polyvinylpyrrolidone, and magnesium metasilicate aluminate), a disintegrant (for example, sodium carboxymethylcellulose), a lubricant (for example, magnesium stearate), a stabilizer, a dissolution adjuvant (glutamic acid, aspartic acid, and the like), and the like, and formulating according to standard methods. As necessary, coating may be carried out with a coating agent (for example, saccharose, gelatin, hydroxypropyl cellulose, and hydroxypropyl methyl cellulose phthalate), and coating of two or more layers may be employed.

Agents for external use as parenteral agents are produced by well-known methods or generally used prescriptions. For example, an ointment may be produced by incorporation or melting of an active ingredient into base material. The ointment base material is selected from well-known material or generally used material. For example, a single material or a mixture of two or more of materials are selected from higher fatty acids and higher fatty acid esters (for example, adipic acid, myristic acid, palmitic acid, stearic acid, oleic acid, adipate esters, myristate esters, palmitate esters, stearate esters, and oleate esters), waxes (for example, beeswax, spermaceti, and ceresin), surfactants (for example, polyoxyethylene alkyl ether phosphate esters), higher alcohols (for example, cetanol, stearyl alcohol, and cetostearyl alcohol), silicone oils (for example, dimethylpolysiloxane), hydrocarbons (for example, hydrophilic petrolatum, white petrolatum, purified lanolin, and liquid paraffin), glycols (for example, ethylene glycol, diethylene glycol, propylene glycol, polyethylene glycol, and macrogol), vegetable oils (for example, castor oil, olive oil, sesame oil, and turpentine oil), animal oils (for example, mink oil, egg yolk oil, squalane, and squalene), water, absorption promoters, and anti-irritants. Furthermore, a humectant, preservative, stabilizer, antioxidant, fragrance, and the like, may be included.

The injection agents as parenteral agents include solutions, suspensions, emulsions and solid injection agents to be dissolved or suspended in a solvent before use. The injection agent is used by, for example, dissolving, suspending or emulsifying an active ingredient in a solvent. Examples of the solvent include distilled water for injection, physiological saline, vegetable oils, alcohols such as propylene glycol, polyethylene glycol, ethanol, and mixtures thereof. Furthermore, the injection agent may contain a stabilizer, a dissolution aid (for example, glutamic acid, aspartic acid, and Polysorbate 80 (registered trademark), etc.), a suspending agent, an emulsifying agent, a soothing agent, a buffer, a preservative, and the like. Such an injection agent is produced by sterilizing at the final step or employing an aseptic process. Furthermore, it is also possible to employ an aseptic solid product such as a freeze-dried product produced and sterilized or dissolved in aseptic distilled water for injection or other solvent before use.

The dose of the Axl inhibitor to be used for the combination of the present invention is different depending on ages, body weights, symptoms, therapeutic effects, administration method, treatment time, and the like. The dose per adult is generally from 1 ng to 1000 mg per dose, once or several times per day by oral administration, or from 0.1 ng to 100 mg per dose, once or several times per day by parenteral administration, or continuous administration for 1 hour to 24 hours per day intravenously. Needless to say, as mentioned above, the dose to be used varies dependent on various conditions. Therefore, dose lower than the ranges specified above may be sufficient in some cases, and dose higher than the ranges specified above are needed in some cases.

When, for example, a compound A is used, a dose in one embodiment is 2 mg/kg to 20 mg/kg body weight, and preferably 2 mg/kg to 20 mg/kg body weight per day.

### (2) Anticancer drug

In the present invention, an anticancer drug means an anticancer drug to be administered for treatment of blood cancer and/or solid cancer. The anticancer drug is not particularly limited as long as it is an anticancer drug to be administered for treatment of blood cancer and/or solid cancer. Examples thereof include cytotoxic anticancer drug, molecular target drug, anti-CTLA-4 antibody, and other anticancer drugs. However, the anticancer drug does not include an immune checkpoint inhibitor other than the anti-CTLA-4 antibody.

In the present invention, the cytotoxic anticancer drug is an anticancer drug having an action to give disorder to cancer cells by inhibiting DNA synthesis of cancer cells or cell division.

Examples of the cytotoxic anticancer drug include alkylating agents, anticancer antibiotics, antimetabolite, plant alkaloid, or platinum preparation.

Examples of the alkylating agent include ifosfamide, cyclophosphamide, dacarbazine, busulfan, procarbazine, bendamustine, melphalan, ranimustine, carmustine, chlorambucil, lomustine, mechlorethamine, nimustine, carboquone, or thiotepa.

Examples of the anticancer antibiotics include idarubicin, epirubicin, daunorubicin, doxorubicin, pirarubicin, bleomycin, mitoxantrone, aclarubicin, mitomycin C, acracinone, zinostatin stimaramer, neocarzinostatin, or pepleomycin.

Examples of the antimetabolite include azacytidine, enocitabine, cladribine, gemcitabine, cytarabine, thioguanine, nelarabine, hydroxyurea, fludarabine, pentostatin, methotrexate, pemetrexed, mercartopurine, decitabine, guadecitabine, CPX-351, clofarabine, hydroxycarbamide, capecitabine, carmofur, tegafur, TS-1, doxifluridine, or fluorouracil.

Examples of the plant alkaloid include irinotecan, etoposide, sobuzoxane, vincristine, vindesine, vinblastine, docetaxel, nogitecan, paclitaxel, or vinorelbine.

Examples of the platinum preparation include carboplatin, cisplatin, nedaplatin, or oxaliplatin.

Among the combinations of the present invention, the cytotoxic anticancer drug to be used for treatment of blood cancer include an alkylating agent, anticancer antibiotics, antimetabolites, plant alkaloids or platinum preparations are preferable, and anticancer antibiotics or antimetabolites are more preferable.

The alkylating agent to be used for treatment of blood cancer is preferably ifosfamide, cyclophosphamide, dacarbazine, busulfan, procarbazine, bendamustine, melphalan, ranimustine, carmustine, chlorambucil, lomustine, mechlorethamine, or nimustine.

The anticancer antibiotics to be used for treatment of blood cancer is preferably idarubicin, epirubicin, daunorubicin, doxorubicin, pirarubicin, bleomycin, mitoxantrone, aclarubicin, or mitomycin C, and more preferably daunorubicin.

The antimetabolite to be used for treatment of blood cancer is preferably azacytidine, enocitabine, cladribine, gemcitabine, cytarabine, thioguanine, nelarabine, hydroxyurea, fludarabine, pentostatin, methotrexate, mercaptopurine, decitabine, CPX-351, clofarabine, or hydroxycarbamide, and more preferably azacytidine or cytarabine.

Examples of the plant alkaloid to be used for treatment of blood cancer is preferably irinotecan, etoposide, sobuzoxane, vincristine, vindesine or vinblastine.

Examples of the platinum preparation to be used for treatment of blood cancer is preferably carboplatin, cisplatin, nedaplatin, or oxaliplatin.

Among the combinations of the present invention, the cytotoxic anticancer drug to be used for treatment of solid cancer include an alkylating agent, antimetabolites, plant alkaloids, a platinum preparation, anticancer antibiotics are preferable, and antimetabolites is more preferable.

The alkylating agent to be used for treatment of solid cancer is preferably ifosfamide, carboquone, cyclophosphamide, dacarbazine, thiotepa, nimustine, melphalan, or ranimustine, and more preferably cyclophosphamide.

The antimetabolite to be used for treatment of solid cancer is preferably capecitabine, carmofur, gemcitabine, cytarabine, tegafur, TS-1, doxifluridine, fluorouracil, or methotrexate, and more preferably gemcitabine.

The plant alkaloid to be used for treatment of solid cancer is preferably irinotecan, etoposide, docetaxel, nogitecan, paclitaxel, vinorelbine, vincristine, vindesine, or vinblastine, and more preferably paclitaxel.

The platinum preparation to be used for treatment of solid cancer is preferably oxaliplatin, carboplatin, cisplatin, or nedaplatin, and more preferably caboplatin.

The anticancer antibiotics to be used for treatment of solid cancer is preferably acracinone, epirubicin, zinostatin stimalamer, doxorubicin, neocarzinostatin, pirarubicin, pepleomycin, or mitomycin C, and more preferably doxorubicin.

In the present invention, the molecular target drug refers to an anticancer agent that targets molecules involved in the proliferation, invasion, or metastasis of cancer cells.

Examples of the molecular target drug to be used for treatment of blood cancer include inhibitors of molecules selected from the group consisting of CD20, CD33, CD52, ABL, proteasome, CCR4, JAK, CXCR4, BET, Bcl-2, HDAC, FLT3, LSD1, MDM2, IDH1, IDH2, Btk, PLK, HSP90, SMO, and NEDD8. The following are examples of the molecular target drug, but the molecular target drugs are note limited thereto.

Examples of the molecular target drug to be used for treatment of blood cancer include an anti-CD20 antibody, an anti-CD33 antibody, an anti-CD52 antibody, an ABL inhibitor, a proteasome inhibitor, an anti-CCR4 antibody, a JAK inhibitor, a CXCR4 antagonistic drug, a BET inhibitor, a Bcl-2 inhibitor, an HDAC inhibitor, an FLT3 inhibitor, an LSD1 inhibitor, an MDM2 inhibitor, an IDH1 inhibitor, an IDH2 inhibitor, a Btk inhibitor, a PLK inhibitor, an HSP90 inhibitor, an SMO inhibitor, or an NEDD8 inhibitor.

Examples of the anti-CD20 antibody include ofatumumab, rituximab, or obinutuzumab.

Examples of the anti-CD33 antibody include Gemtuzumab or Gemtuzumab ozogamicin.

Examples of the anti-CD52 antibody include alemtuzumab.

Examples of the ABL inhibitor include imatinib, dasatinib, or nilotinib.

Examples of the proteasome inhibitor include bortezomib, carfilzomib, and ixazomib.

Examples of the anti-CCR4 antibody include mogamulizumab.

Examples of the JAK inhibitor include ruxolitinib.

Examples of the CXCR4 antagonistic drug include AMD3100, BMS-936564, BL-8040, Dociparstat sodium, or LY2624587 (a CXCR4 inhibitory antibody).

Examples of the BET inhibitor include OTX015, GSK525762, RVX-208, BMS-986158, PLX51107, CPI-0610, TEN-010, INCB054329, ABBV075, or GS-5829.

Examples of the Bcl-2 inhibitor include ABT-199 (venetoclax), ABT-263, GX15-070, orAT-101.

Examples of the HDAC inhibitor include pracinostat, vorinostat, romidepsin, panobinostat, belinostat, entinostat, or chidamide.

Examples of the FLT3 inhibitor include gilteritinib, midostaurin, sorafenib, ponatinib, crenolanib, tandutinib, sunitinib, quizartinib, or pacritinib.

Examples of the LSD1 inhibitor include GSK-2879552, INCB-59872, or ORY-1001.

Examples of the MDM2 inhibitor include idasanutlin, SAR405838, DS-3032b, RG7112, HDM201, MK4828, AMG-232, or ALRN-6924.

Examples of the IDH1 inhibitor include ivosidenib.

Examples of theIDH2 inhibitor include enasidenib.

Examples of the Btk inhibitor include ONO-4059 (tirabrutinib), ibrutinib, spebrutinib, HM-71224 (LY3337641), acalabrutinib, SNS-062, BGB-311, GDC-0853, M2951, BMS-986142, PRN-1008, TAK-020, TAS5315, pharmacyclics-3, or AC-0058TA.

Examples of the PLK inhibitor include volasertib, GSK461364, rigosertib, BI2536, HMN-176, NMS-P937, CYC-140, or RO3280.

Examples of the HSP90 inhibitor include ganetespib.

Examples of the SMO inhibitor include glasdegib, vismodegib, or sonidegib.

Examples of the NEDD8 inhibitor include pevonedistat.

Examples of the molecular target drug to be used for treatment of solid cancer include an EGFR inhibitor, a Bcr-Abl inhibitor, a VEGFR inhibitor, an mTOR inhibitor, an ALK inhibitor, a PARP inhibitor, a BRAF inhibitor, an MEK inhibitor, a CDK inhibitor, an HER2 inhibitor, a multikinase inhibitor, a proteasome inhibitor, a Bcl-2 inhibitor, an HDAC inhibitor, or the like.

Examples of the EGFR inhibitor include gefitinib, erlotinib, afatinib, cetuximab, dacomitinib, or panitumumab.

Examples of the Bcr-Abl inhibitor include imatinib.

Examples of the VEGFR inhibitor include bevacizumab, or pazopanib.

Examples of the mTOR inhibitor include everolimus, or temsirolimus.

Examples of the ALK inhibitor include ceritinib, or alectinib.

Examples of the PARP inhibitor include olaparib, rucaparib, or niraparib.

Examples of the BRAF inhibitor include vemurafenib, dabrafenib, or encorafenib.

Examples of the MEK inhibitor include trametinib, selumetinib, binimetinib, or CH4987655.

Examples of the CDK inhibitor include palbociclib or dinaciclib.

Examples of the HER2 inhibitor include lapatinib or trastuzumab.

Examples of the multikinase inhibitor include regorafenib, sunitinib, vandetanib, crizotinib, or sorafenib. Note here that sunitinib and sorafenib are also examples of the FLT3 inhibitor.

Examples of the proteasome inhibitor include bortezomib.

Examples of the HDAC inhibitor include panobinostat.

Among the combinations of the present invention, the molecular target drug to be used for treatment of blood cancer is preferably an anti-CD20 antibody, an anti-CD33 antibody, an anti-CD52 antibody, an ABL inhibitor, a proteasome inhibitor, an anti-CCR4 antibody, a JAK inhibitor, a CXCR4 antagonistic drug, a BET inhibitor, a Bcl-2 inhibitor, an HDAC inhibitor, an FLT3 inhibitor, an LSD1 inhibitor, an MDM2 inhibitor, an IDH1 inhibitor, an IDH2 inhibitor, a Btk inhibitor, or a PLK inhibitor, and more preferably a CXCR4 antagonistic drug, a BET inhibitor, a Bcl-2 inhibitor, an HDAC inhibitor, an FLT3 inhibitor, an LSD1 inhibitor, an MDM2 inhibitor, a Btk inhibitor, or a PLK inhibitor.

The anti-CD20 antibody is preferably ofatumumab, rituximab, or obinutuzumab.

The anti-CD33 antibody is preferably gemtuzumab or gemtuzumab ozogamicin.

The anti-CD52 antibody is preferably alemtuzumab.

The ABL inhibitor is preferably imatinib, dasatinib, or nilotinib.

The proteasome inhibitor is preferably bortezomib, carfilzomib, or ixazomib.

The anti-CCR4 antibody is preferably mogamulizumab.

The JAK inhibitor is preferably ruxolitinib.

The CXCR4 antagonistic drug is preferably AMD3100, BMS-936564, BL-8040, dociparstat sodium or LY2624587, and more preferably AMD3100.

The BET inhibitor is preferably ABBV-075, GSK525762, RVX-208, BMS-986158, PLX51107, CPI-0610, TEN-010, INCB054329, or GS-5829, and more preferably ABBV-075.

The Bcl-2 inhibitor is preferably ABT-199 (venetoclax), ABT-263, GX15-070, or AT-101, and more preferably ABT-199 (venetoclax).

The HDAC inhibitor is preferably plasinostat, vorinostat, romidepsin, panobinostat, belinostat, entinostat, or chidamide, and more preferably plasinostat.

The FLT3 inhibitor is preferably gilteritinib, midostaurin, sorafenib, ponatinib, crenolanib, tandutinib, sunitinib, quizartinib, or pacritinib, and more preferably gilteritinib or midostaurin.

The LSD1 inhibitor is preferably GSK-2879552, INCB-59872, or ORY-1001, and more preferably GSK-2879552.

The MDM2 inhibitor is preferably idasanutlin, SAR405838, DS-3032b, RG7112, HDM201, MK4828, AMG-232, or ALRN-6924, and more preferably idasanutlin.

IDH1 inhibitor is preferably ivosidenib.

IDH2 inhibitor is preferably enasidenib.

Btk inhibitor is preferably ONO-4059 (tirabrutinib), ibrutinib, spebrutinib, HM-71224, Acalabrutinib, SNS-062, BGB-311, GDC-0853, M2951, BMS-986142, PRN-1008, TAK-020, TAS5315, pharmacyclics-3, or AC-0058TA, and more preferably ONO-4059 (tirabrutinib).

The PLK inhibitor is preferably volasertib, GSK461364, rigosertib, BI2536, HMN-176, NMS-P937, CYC-140, or RO3280, and more preferably volasertib.

Among the combinations of the present invention, the molecular target drug to be used for treatment of solid cancer is preferably an EGFR inhibitor, a Bcr-Abl inhibitor, a VEGFR inhibitor, an mTOR inhibitor, an ALK inhibitor, a PARP inhibitor, a BRAF inhibitor, an MEK inhibitor, a CDK inhibitor, an HER2 inhibitor, a multikinase inhibitor, a proteasome inhibitor, a Bcl-2 inhibitor, or an HDAC inhibitor.

The EGFR inhibitor is preferably gefitinib, erlotinib, afatinib, cetuximab, or panitumumab, and more preferably gefitinib, erlotinib, or afatinib.

The Bcr-Abl inhibitor is preferably imatinib.

The VEGFR inhibitor is preferably bevacizumab or pazopanib, and more preferably bevacizumab.

The mTOR inhibitor is preferably everolimus or temsirolimus.

The ALK inhibitor is preferably ceritinib or alectinib.

The PARP inhibitor is preferably olaparib, rucaparib, or niraparib.

The BRAF inhibitor is preferably vemurafenib, dabrafenib, or encorafenib, and more preferably vemurafenib or dabrafenib.

The MEK inhibitor is preferably trametinib, selumetinib, binimetinib, or CH4987655, and more preferably trametinib.

The CDK inhibitor is preferably palbociclib, or dinaciclib.

The HER2 inhibitor is preferably lapatinib, or trastuzumab.

The multikinase inhibitor is preferably regorafenib, sunitinib, vandetanib, crizotinib, or Sorafenib.

The proteasome inhibitor is preferably bortezomib.

The Bcl-2 inhibitor is preferably venetoclax.

The HDAC inhibitor is preferably panobinostat.

In the present invention, CTLA-4(cytotoxic T lymphocyte antigen-4) is a member of protein immunoglobulin superfamily. CTLA-4 downregulates the activation of the T-cells to exhibit an immunosuppression function. Therefore, the anti-CTLA-4 antibody suppresses the function (signal) of CTLA-4, and thereby releases the immunosuppression function, and the immune response to cancer can be activated. Examples of the anti-CTLA-4 antibody to be used for the combination of the present invention include ipilimumab (YERVOY (registered trademark)), tremelimumab, or AGEN-1884. Furthermore, antibodies including heavy chain and light chain complementarity determining regions (CDRs) or variable region (VR) of the above-mentioned known anti-CTLA-4 antibody are also one embodiment of the anti-CTLA-4 antibody. Examples of further embodiment of the anti-CTLA-4 antibody include an antibody including heavy chain and light chain complementarity determining regions (CDRs) or variable region (VR) of ipilimumab.

The dose of the anti-CTLA-4 antibody to be used for the combination of the present invention is different depending on ages, body weights, symptoms, therapeutic effects, administration methods, treatment time, and the like, but is adjusted such that the optimum desired effect can be exhibited.

When the anti-CTLA-4 antibody is used, the dose in one embodiment is 0.1 to 20 mg/kg body weight. Furthermore, when an antibody (for example, ipilimumab) including the heavy chain and light chain complementarity determining regions (CDRs) or the variable region (VR) of ipilimumab is used, the dose in one embodiment is 0.3 to 10 mg/kg body weight, and preferably 3 mg/kg body weight.

In the present invention, the other anticancer drugs mean anticancer drugs other than the cytotoxic anticancer drug and the molecular target drug to be used for treatment of blood cancer. Examples of the anticancer drug for treatment of blood cancer include a steroid preparation, a vitamin A derivative, an antiviral agent, L-asparaginase, arsenite, interferon-alpha, cyclosporine, thalidomide, pomalidomide, and lenalidomide.

Examples of the steroid preparation include dexamethasone, prednisolone, or methylprednisolone.

Examples of the vitamin A derivative include all-trans retinoic acid or tamibarotene.

Examples of the antiviral agent include acyclovir or zidovudine.

The other anticancer drugs to be used for treatment of blood cancer in the combination of the present invention are preferably a steroid preparation, a vitamin A derivative, an antiviral agent, L-asparaginase, arsenite, interferon α, cyclosporine, thalidomide, pomalidomide, or lenalidomide, and more preferably a steroid preparation.

The steroid preparations are preferably dexamethasone, prednisolone, or methylprednisolone, and more preferably dexamethasone.

The vitamin A derivative is preferably all-trans retinoic acid or tamibarotene.

The antiviral agent is preferably acyclovir or zidovudine.

In the present invention, the other anticancer drugs mean anticancer drugs excluding the cytotoxic anticancer drug and the molecular target drug among the anticancer drugs to be administered for treatment of solid cancer.

Examples of the other anticancer drugs for treatment of solid cancer include steroid, a hormone preparation, an aromatase inhibitor, or interferon α, and the like.

As the other anticancer drugs for treatment of solid cancer to be used in combination in the present invention, steroid, a hormone preparation, an aromatase inhibitor, interferon α are preferable, and a hormone preparation and an aromatase inhibitor are more preferable.

The steroid is preferably dexamethasone, prednisolone, or methylprednisolone, and more preferably dexamethasone.

The hormone preparation is preferably medroxyprogesterone, methyl testosterone, ethynyl estradiol, goserelin, leuprorelin, chlormadinone, flutamide, bicalutamide, tamoxifen, toremifene, or mepitiostane, and more preferably ethynyl estradiol.

The aromatase inhibitor is preferably anastrozole, exemestane, or letrozole, and more preferably letrozole.

Furthermore, as the combination of the present invention, cell-based therapy (for example, chimeric antigen receptor T-cell (CAR-T) therapy) may be combined.

Among these anticancer drugs, one or a plurality of arbitrary types may be combined with the Axl inhibitor according to the present invention and can be used for cancer treatment.

The dose of the anticancer drug to be used in the combination of the present invention is different depending on ages, body weights, symptoms, therapeutic effects, administration methods, treatment time, and the like, but is adjusted such that the optimum desired effect can be exhibited.

### [Toxicity]

The combination of the present invention has sufficiently low toxicity, and, thereof, it can be used safely as pharmaceutical preparations.

### [Application to pharmaceutical preparations]

One embodiment of diseases to be treated by the combination of the present invention or a compound A as an Axl inhibitor include a blood cancer. The blood cancer is not particularly limited, and examples of the blood cancer include leukemia, malignant lymphoma, and myeloma.

Examples of leukemia include acute myeloid leukemia, acute promyelocytic leukemia, acute lymphoblastic leukemia (acute lymphatic leukemia), lymphoblastic lymphoma, chronic myeloid leukemia, myeloproliferative neoplasm, chronic lymphatic leukemia, small lymphocytic lymphoma, and myelodysplastic syndrome.

Examples of acute myeloid leukemia includes acute megakaryoblastic leukemia (AML-M7 or FAB subtype-M7).

Examples of malignant lymphoma include follicular lymphoma, MALT lymphoma, marginal zone lymphoma, lymphoplasmacytic lymphoma, mantle-cell lymphoma, diffuse large B-cell lymphoma, Burkitt lymphoma, peripheral T-cell lymphoma, adult T-cell leukemia/lymphoma, extranodal NK/T cell lymphoma, nasal type and Hodgkin lymphoma.

Examples of myeloma include multiple myeloma and related disease of multiple myeloma.

One embodiment of diseases treated by the combination of the present invention is solid cancer. The solid cancer are not particularly limited, but examples thereof include head and neck cancer, nasopharyngeal cancer, esophageal cancer, gastro-esophageal junction cancer, esophageal adenocarcinoma, stomach cancer, large-intestine cancer, colon cancer, rectum cancer, small-intestine cancer, anal cancer (for example, anal canal cancer), liver cancer (for example, hepatocellular carcinoma), gallbladder cancer, bile duct cancer, biliary tract cancer, pancreatic cancer, thyroid cancer, parathyroid cancer, lung cancer (for example, non-small cell lung cancer (for example, squamous non-small cell lung cancer, non-squamous non-small cell lung cancer), small cell lung cancer), breast cancer, ovarian cancer (for example, serous ovarian cancer, ovarian clear cell adenocarcinoma), fallopian tube cancer, uterine cancer (for example, cervical cancer, uterine body cancer, endometrial cancer), vaginal cancer, vulvar cancer, penile cancer, kidney cancer (for example, renal cell carcinoma, clear cell renal cell carcinoma), adrenal cancer, urothelial carcinoma (for example, urinary bladder cancer, upper urinary tract cancer, ureteral cancer, renal pelvic cancer, and urethral cancer), prostate cancer, testicular tumor (for example, germ cell tumor), bone and soft tissue sarcoma (for example, Ewing's sarcoma, childhood rhabdomyosarcoma, and uterine body leiomyosarcoma), skin cancer (for example, uveal malignant melanoma, malignant melanoma (for example, malignant melanoma in the skin, oral mucoepithelium or intraorbital, etc.), Merkel cell carcinoma), glioma (for example, glioblastoma, gliosarcoma), brain tumor (for example, glioblastoma), spine tumor, Kaposi's sarcoma, squamous cell carcinoma, pleural mesothelioma, primary peritoneal cancer, endocrine cancer, childhood cancer, or cancer of unknown primary.

Among them, for example, in patients who have a blood cancer and/or a solid cancer and cannot obtain sufficient therapeutic effect by a single use of the anticancer drug or the Axl inhibitor, it is expected that the combination of the present invention can exhibit the maximum antitumor effect. Furthermore, the combination of the present invention can reduce the dose of each of the drugs. As a result, reduction of the adverse reaction can be expected.

Lung cancer in the present invention includes EGFR gene exon 19 deletion mutation positive lung cancer. EGFR exon 19 deletion mutation positive lung cancer is lung cancer that is EGFR gene mutation positive.

Malignant melanoma of the present invention includes BRAF gene activating mutation positive malignant melanoma. The BRAF gene activating mutation positive malignant melanoma is malignant melanoma having BRAF gene mutation.

Patients who have solid cancer and cannot obtain sufficient therapeutic effect by the Axl inhibitor in the present invention includes: (1) patients having solid cancer that is refractory to the Axl inhibitor, or (2) solid cancer patients who have progressed during or after treatment with the Axl inhibitor.

Patients who have solid cancer and cannot obtain sufficient therapeutic effect by the anticancer drug in the present invention includes: (1) patients with solid cancer that is refractory to the anticancer drug, or (2) solid cancer patients who have progressed during or after treatment with the anticancer drug.

Examples of the solid cancer that is refractory to the anticancer drug of the present invention include gemcitabine-resistant pancreatic cancer, EGFR inhibitor-resistant lung cancer (for example, gefitinib-resistant lung cancer), BRAF inhibitor-resistant malignant melanoma (for example, vemurafenib-resistant malignant melanoma), but it is not limited thereto.

In one embodiment, the combination of the present invention can be applied to treatment of metastatic carcinoma or suppression of metastasis.

In one embodiment, the combination of the present invention suppresses relapse.

In the present invention, treatment means bringing about at least one effect of extension of progression-free survival time (PFS), extension of overall survival time (OS), extension of disease-free survival time (DFS), extension of progression-free period (TTP), extension of event-free survival (EFS), extension of relapse-free survival (RFS), reduction of the number of cancer cells, reduction of a tumor size, suppression of tumor growth (retardation or stopping), suppression of tumor metastasis (retardation or stopping), suppression of the recurrence (prevention or retardation), and alleviation of one or a plurality of symptoms associated with cancer.

The "administering in combination" in the present invention includes simultaneous administration of compounds having the same or different dosage form, or administration of compounds separately (for example, sequential administration). More specifically the "administering in combination" includes administering a form of a compounding agent including all components blended in one formulation, or administering as separate formulations. Administration as separate formulations includes simultaneous administration and administration at different times. In the administration at different times, an Axl inhibitor may be administered before anticancer drugs. Alternatively, the anticancer drugs may be administered before the Axl inhibitor. The method for the administration of these drugs may be the same as or different from each other.

In the present invention, the combination of the present invention may be administered in combination with other medicine (for example, well-known agents for cancer treatment) for the purposes of: (1) supplementing and/or enhancing therapeutic effect, (2) improving the kinetics, improving absorption, and reducing the dose; and/or (3) eliminating the adverse reaction of the compound.

In the present invention, patients "treated with an anticancer drug" and patients "treated with the compound represented by the general formula (I), a salt thereof, a solvate thereof, an N-oxide thereof, or a prodrug thereof described in the above [1]" means both patients treated with "the anticancer drug" or "the compound represented by the general formula (I), a salt thereof, a solvate thereof, an N-oxide thereof, or a prodrug thereof according to the above [1]" before treatment with the other drug of the combination of the present invention, and patients treated with "the anticancer drug" or "the compound represented by the general formula (I), a salt thereof, a solvate thereof, an N-oxide thereof, or a prodrug thereof according to the above [1]" during treatment with the other drug of the combination of the present invention.

The present application provides, for example, the following embodiments.
[1] An agent for cancer treatment, including an Axl inhibitor as an active ingredient, which is administered in combination with an anticancer drug, wherein the Axl inhibitor is a compound represented by the general formula (I): 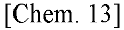 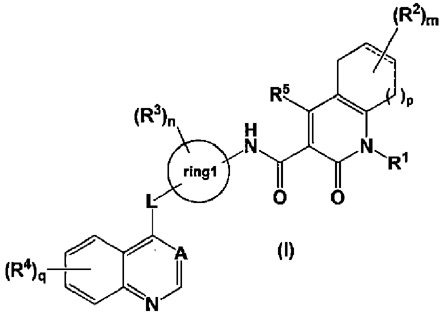
   [wherein R¹ represents (1) a C1-8 alkyl group optionally substituted with one to five R¹¹, (2) a C3-7 carbocyclic ring optionally substituted with one to five R¹², or (3) a 4- to 7-membered heterocycle optionally substituted with one to five R¹³, and herein when the C1-8 alkyl group represented by R¹ is a branched alkyl group, C1-3 alkyl groups branched from the same carbon atom, together with carbon atom bonded thereto, may form a saturated C3-7 carbocyclic ring;
   R² represents (1) a C1-4 alkyl group, (2) a halogen atom, (3) a C1-4 haloalkyl group, (4) an oxo group, (5) an -OR²¹ group, or (6) an =NR²² group;
   R³ represents (1) a C1-4 alkyl group, (2) a halogen atom, or (3) a C1-4 haloalkyl group;
   R⁴ represents (1) a C1-4 alkoxy group, (2) a C1-4 haloalkyl group, or (3) an -OR⁴¹ group, (4) a C1-4 alkyl group, (5) a C2-4 alkenyloxy group, or (6) a C2-4 alkynyloxy group;
   R⁵ represents (1) a hydrogen atom, (2) a C1-4 alkyl group, (3) a halogen atom, (4) a C1-4 haloalkyl group, or (5) an -OR²¹ group;
   R¹¹ represents (1) an -OR¹⁰¹ group, (2) an SO₂R¹⁰² group, (3) an NR¹⁰³R¹⁰⁴ group, or (4) a C3-7 carbocyclic ring optionally substituted with one to three halogen atoms;
   R¹² represents (1) a C1-8 alkyl group optionally substituted with a hydroxyl group, or (2) a halogen atom;
   R¹³ represents (1) a C1-8 alkyl group optionally substituted with a hydroxyl group, or (2) a halogen atom;
   R²¹ represents (1) a hydrogen atom, or (2) a C1-4 alkyl group;
   R²² represents (1) a hydroxyl group, or (2) a C1-4 alkoxy group;
   R⁴¹ represents (1) a hydrogen atom,
   (2) a C1-8 alkyl group substituted with one or two substituents selected from the group consisting of (a) a 5- to 7-cyclic group optionally substituted with one to two substituents selected from the group consisting of (i) a C1-4 alkyl group, (ii) a C1-4 haloalkyl group, and (iii) a halogen atom, (b) NR⁴⁰¹R⁴⁰², (c) a hydroxyl group, and (d) a SO₂R⁴⁰³ group;
   (3) a C2-8 alkenyl group substituted with one or two substituents selected from the group consisting of (a) a 5- to 7-cyclic group optionally substituted with one or two substituents selected from the group consisting of (i) a C1-4 alkyl group, (ii) a C1-4 haloalkyl group, and (iii) a halogen atom, (b) NR⁴⁰¹R⁴⁰², (c) a hydroxyl group, and (d) an SO₂R⁴⁰³ group; or
   (4) a C2-8 alkynyl group substituted with one or two substituents selected from the group consisting of (a) a 5- to 7-cyclic group optionally substituted with one or two substituents selected from the group consisting of (i) a C1-4 alkyl group, (ii) a C1-4 haloalkyl group, and (iii) a halogen atom, (b) NR⁴⁰¹R⁴⁰², (c) a hydroxyl group, and (d) an SO₂R⁴⁰³ group,
   R¹⁰¹ represents (1) a hydrogen atom, or (2) a C1-4 alkyl group;
   R¹⁰² represents (1) a hydrogen atom, or (2) a C1-4 alkyl group;
   R¹⁰³ and R¹⁰⁴ each independently represent (1) a hydrogen atom, or (2) a C1-4 alkyl group;
   R⁴⁰¹ and R⁴⁰² each independently represent (1) a hydrogen atom, or (2) a C1-4 alkyl group;
   R⁴⁰³ represents (1) a hydrogen atom, or (2) a C1-4 alkyl group;
   A represents (1) CH, or (2) a nitrogen atom;
   L represents (1) -O-, (2) -NH-, (3) -C(O)-, (4) -CR⁶R⁷-, (5) -S-, (6) -S(O)-, or (7) -S(O)₂-;
   R⁶ and R⁷ each independently represent (1) a hydrogen atom, (2) a halogen atom, (3) a C1-4 alkyl group, (4) a hydroxyl group, or (5) NH₂;
   ring1 represents a 5- to 7-membered cyclic group; 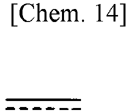
   represents a single bond, or a double bond;
   m represents an integer of 0 to 5;
   n represents an integer of 0 to 5;
   p represents an integer of 0 to 2;
   q represents an integer of 0 to 4;
   when m is 2 or more, a plurality of R²'s may be the same as or different from each other, and herein when two R²'s represent a C1-3 alkyl group and are on the same carbon atom, the R²'s together with a carbon atom bonded thereto, may form a C3-7 saturated carbocyclic ring; when n is 2 or more, a plurality of R³'s may be the same as or different from each other; and when q is 2 or more, a plurality of R⁴'s may be the same as or different from each other], a salt thereof, a solvate thereof, an N-oxide thereof, or a prodrug thereof (wherein the anticancer drug does not include an immune checkpoint inhibitor other than an anti-CTLA-4 antibody),
[2] The agent according to the above [1], wherein the Axl inhibitor is a compound represented by the general formula (I-1): 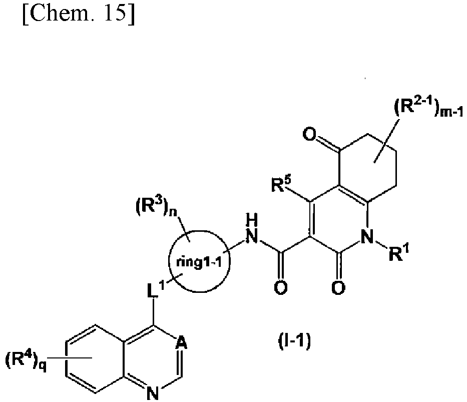
   [wherein in the formula, R²⁻¹ represents (1) a C1-4 alkyl group, (2) a halogen atom, (3) a C1-4 haloalkyl group, (4) an -OR²¹ group, or (5) a =NR²² group; m-1 represents an integer of 0 to 4;
   L¹ represents (1) -O-, (2) -NH-, or (3) -C(O)-;
   ring1-1 represents benzene or pyridine;
   when m-1 is 2 or more, a plurality of R²⁻¹'s may be the same as or different from each other, and herein when the two R²⁻¹'s represent a C1-3 alkyl group and are on the same carbon atom, the R²⁻¹'s together with carbon atom bonded thereto, may form a C3-7 saturated carbocyclic ring; and
   the other symbols have the same meanings as defined above], a salt thereof, a solvate thereof, an N-oxide thereof, or a prodrug thereof,
[3] the agent according to the above [1] or [2], wherein the Axl inhibitor is N-{5-[(6,7-dimethoxy-4-quinolinyl)oxy]-2-pyridinyl}-2,5-dioxo-1-phenyl-1,2,5,6,7,8-hexahydro-3-quinolinecarboxamide, a pharmaceutically acceptable salt thereof or a hydrate thereof,
[4] the agent according to any one of the above [1] to [3], wherein the cancer is a blood cancer,
[5] the agent according to the above [4], wherein the blood cancer is leukemia,
[6] the agent according to the above [5], wherein the leukemia is acute myeloid leukemia or acute lymphoblastic leukemia,
[7] the agent according to any one of the above [4] to [6], wherein the anticancer drug is selected from the group consisting of a cytotoxic anticancer drug, a molecular target drug, a steroid preparation, a vitamin A derivative, an antiviral agent, L-asparaginase, arsenite, interferon α, cyclosporine, thalidomide, pomalidomide, and lenalidomide,
[8] the agent according to the above [7], wherein the anticancer drug is a cytotoxic anticancer drug,
[9] the agent according to the above [8], wherein the cytotoxic anticancer drug is selected from the group consisting of daunorubicin, azacytidine, and cytarabine,
[10] the agent according to the above [7], wherein the anticancer drug is a molecular target drug,
[11] the agent according to the above [10], wherein the molecular target drug is selected from the group consisting of an anti-CD20 antibody, an anti-CD33 antibody, an anti-CD52 antibody, an ABL inhibitor, a proteasome inhibitor, an anti-CCR4 antibody, a JAK inhibitor, a CXCR4 antagonistic drug, a BET inhibitor, a Bcl-2 inhibitor, an HDAC inhibitor, an FLT3 inhibitor, an LSD1 inhibitor, an MDM2 inhibitor, an IDH1 inhibitor, an IDH2 inhibitor, a Btk inhibitor, a PLK inhibitor, an HSP90 inhibitor, an SMO inhibitor, and NEDD8 inhibitor,
[12] the agent according to the above [10] or [11], wherein the molecular target drug is selected from the group consisting of a CXCR4 antagonistic drug, a BET inhibitor, a Bcl-2 inhibitor, an HDAC inhibitor, an FLT3 inhibitor, an LSD1 inhibitor, an MDM2 inhibitor, a Btk inhibitor, and a PLK inhibitor,
[13] the agent according to any one of the above [10] to [12], wherein the molecular target drug is selected from the group consisting of AMD3100, ABBV-075, ABT-199, plasinostat, gilteritinib, midostaurin, GSK-2879552, idasanutlin, tirabrutinib, and volasertib,
[14] the agent according to any one of the above [1] to [3], wherein the cancer is solid cancer,
[15] the agent according to the above [14], wherein the solid cancer is head and neck cancer, nasopharyngeal cancer, esophageal cancer, gastro-esophageal junction cancer, esophageal adenocarcinoma, stomach cancer, large-intestine cancer, colon cancer, rectum cancer, small-intestine cancer, anal cancer, liver cancer, gallbladder cancer, bile duct cancer, biliary tract cancer, pancreatic cancer, thyroid cancer, parathyroid cancer, lung cancer, breast cancer, ovarian cancer, fallopian tube cancer, uterine cancer, vaginal cancer, vulvar cancer, penile cancer, kidney cancer, adrenal cancer, urothelial carcinoma, prostate cancer, testicular tumor, bone and soft tissue sarcoma, skin cancer, glioma, brain tumor, spine tumor, Kaposi's sarcoma, squamous cell carcinoma, pleural mesothelioma, primary peritoneal cancer, endocrine cancer, childhood cancer, or cancer of unknown primary,
[16] the agent according to the above [14] or [15], wherein the solid cancer is pancreatic cancer, lung cancer, or skin cancer,
[17] the agent according to any one of the above [14] to [16], wherein the anticancer drug is selected from the group consisting of a cytotoxic anticancer drug, a molecular target drug, steroid, a hormone preparation, an aromatase inhibitor, and interferon α,
[18] the agent according to the above [17], wherein the anticancer drug is a cytotoxic anticancer drug,
[19] the agent according to the above [18], wherein the cytotoxic anticancer drug is an alkylating agent, antimetabolite, a plant alkaloid drug, a platinum preparation, and anticancer antibiotics,
[20] the agent according to the above [19], wherein the cytotoxic anticancer drug is an antimetabolite,
[21] the agent according to the above [20], wherein the antimetabolite is gemcitabine,
[22] the agent according to the above [17], wherein the anticancer drug is a molecular target drug,
[23] the agent according to the above [22], wherein the molecular target drug is selected from the group consisting of an EGFR inhibitor, a Bcr-Abl inhibitor, a VEGFR inhibitor, an mTOR inhibitor, an ALK inhibitor, a PARP inhibitor, a BRAF inhibitor, an MEK inhibitor, a CDK inhibitor, an HER2 inhibitor, a multikinase inhibitor, a proteasome inhibitor, a Bcl-2 inhibitor, and an HDACβ inhibitor,
[24] the agent according to the above [22] or [23], wherein the molecular target drug is selected from the group consisting of the EGFR inhibitor, the BRAF inhibitor, and the MEK inhibitor,
[25] the agent according to the above [24], wherein the EGFR inhibitor is gefitinib, erlotinib, or afatinib, the BRAF inhibitor is vemurafenib or dabrafenib, and the MEK inhibitor is trametinib,
[26] the agent according to any one of the above [1] to [3], wherein the anticancer drug is an anti-CTLA-4 antibody,
[27] the agent according to the above [26], wherein the anti-CTLA-4 antibody is selected from the group consisting of ipilimumab, tremelimumab, and AGEN-1884,
[28] a method for treating cancer, the method including administering an effective amount of a compound represented by the general formula (I), a salt thereof, a solvate thereof, an N-oxide thereof, or a prodrug thereof according to the above [1] in combination with an anticancer drug to a patient in need of treatment of cancer (wherein the anticancer drug does not include an immune checkpoint inhibitor other than an anti-CTLA-4 antibody),
[29] a compound represented by the general formula (I), a salt thereof, a solvate thereof, an N-oxide thereof, or a prodrug thereof according to the above [1], to be used in combination with an anticancer drug for cancer treatment (wherein the anticancer drug does not include an immune checkpoint inhibitor other than an anti-CTLA-4 antibody),
[30] Use of a compound represented by the general formula (I), a salt thereof, a solvate thereof, an N-oxide thereof, or a prodrug thereof according to the above [1], for the manufacture of an agent for cancer treatment, wherein the agent for cancer treatment is administered in combination with an anticancer drug (wherein the anticancer drug does not include an immune checkpoint inhibitor other than an anti-CTLA-4 antibody),
[31] an agent for cancer treatment, containing an anticancer drug as an active ingredient, which is administered in combination with an Axl inhibitor, wherein the Axl inhibitor is a compound represented by the general formula (I), a salt thereof, a solvate thereof, an N-oxide thereof, or a prodrug thereof according to the above [1] (wherein the anticancer drug does not include an immune checkpoint inhibitor other than an anti-CTLA-4 antibody),
[32] a pharmaceutical composition for cancer treatment, which is administered in combination with an Axl inhibitor and an anticancer drug, wherein the Axl inhibitor is a compound represented by the general formula (I), a salt thereof, a solvate thereof, an N-oxide thereof, or a prodrug thereof according to the above [1] (wherein the anticancer drug does not include an immune checkpoint inhibitor other than an anti-CTLA-4 antibody), and the like.

### [EXAMPLES]

Hereinafter, the present invention is described specifically with reference to Examples, but the present invention is not limited thereto.

As an Axl inhibitor represented by the general formula (I), N-{5-[(6,7-dimethoxy-4-quinolinyl)oxy]-2-pyridinyl}-2,5-dioxo-1-phenyl-1,2,5,6,7,8-hexahydro-3-quinolinecarboxamide (a compound A) was used. The compound A can be produced by a well-known method, for example, a method described in Example 5 of WO2015/012298.

Combination index used in the synergistic analysis of the present application is represented by Ci or CI.

### Biological Example 1: Reduction test or proliferation test by administration singly or in combination of compound A and various anticancer drugs using sample of bone marrow derived from patient with acute myeloid leukemia

Initial evaluation: Bone marrow samples were obtained from an adult acute myeloid leukemia patient. To determine the best marker combination for identifying leukemia cells in each sample, a very small amount of samples were stained with a monoclonal antibody (MAb) specific to the various markers. Furthermore, to evaluate the initial survival rate of cells, Annexin V was added to the above-mentioned combination of MAb. The combinations of Mab used in the initial evaluation were shown in the following table.

**[Table 1]**

| Combination of Annexin V or MAb and coloring agent | | | | | | |
|---|---|---|---|---|---|---|
| 1 | ANEX | CD34 | CD45 | HLADR | CD117 | CD19 |
| | FITC | PerCP | PO | PE | APC | PECy7 |
| 2 | ANEX | CD34 | CD45 | CD64 | CD14 | CD33 |
| | FITC | PerCP | PO | PE | APC | PB |
| 3 | ANEX | CD34 | CD45 | CD13 | CD11b | |
| | FITC | PerCP | PO | PE | APC | |

Reduction test: Whole bone marrow sample including leukemia cells was diluted with a medium (RPMI1640 containing 20% FBS, 2% HEPES, 1% antibiotics, and 1% L-glutamine) (hereinafter, referred to as a medium 1), and dispensed into a 96-well plate containing a DMSO solution of a drug, which had been prepared in advance (final volume: 60 µL/well, DMSO concentration: 0.5% or less). The plate containing the sample was incubated at 37°C in humidified air including 5% CO₂ for 96 hours. In order to prepare a sample for analysis, erythrocytes were lysed after completion of incubation, and then, the sample was stained with the best combination of Mab and Annexin V to identify the leukemia cells in the sample. Finally, the plate was analyzed by ExviTech (registered trademark) platform.

Proliferation test: Proliferation was measured using a CFDA-SE coloring agent. A coloring agent which had been prepared in advance was placed in 15-mL tube including bone marrow enough to obtain about 6000 cells/well of living leukemia cells. Thereafter, they were diluted with the medium such that the total volume became 1 mL. After the resultant product was mixed well by vortex, the mixture was incubated on a rotator at room temperature with light shielded for 10 minutes. Then, the tube was filled with cool RPMI1640 including 10% FBS (medium 2), and incubated on ice for 5 minutes to stop the reaction. Thereafter, the cells were washed with the medium 2 twice. For the final resuspension before plating and incubation, the medium 1 replenished with a cytokine cocktail was used. The suspension was dispensed into a 96-well plate containing a DMSO solution of the drug which had been prepared in advance (final volume: 60 µL/well, DMSO concentration: 0.5% or less). The plate containing the sample was incubated at 37°C in humidified air including 5% CO₂ for 96 hours. In order to prepare a sample for analysis, erythrocytes were lysed after completion of incubation, and then, the sample was stained with the best combination of Mab and Annexin V to identify the leukemia cells in the sample. Finally, the plate was analyzed by ExviTech (registered trademark) platform.

Data analysis: Synergistic analysis was carried out by calculating Combination Index (Ci) as described in Cancer Research, Vol. 70, No. 2, pp. 440-446, 2010. This analysis method is the easiest and simplest method for measuring the drug interaction. Note here that the meaning of the Ci value is regarded as follows.
Ci<1: synergistic action, Ci=1: additive action, Ci>1: antagonistic action

Result of reduction test: Table 2 shows median values of Ci calculated with respect to the compound A and various anticancer drugs. The median values of Ci when the compound A and OTX015, AMD3100, plasinostat, daunorubicin, ABT-199, idasanutlin or cytarabine were used in combination were less than 1, revealing that the combined use of the compound A and these drugs showed the synergistic action. From the above, it was verified that the combined use of the compound A and these drugs exhibited a strong antitumor effect.

**[Table 2]**

| Drug to be combined | Ci median value in combined use with compound A |
|---|---|
| OTX015 | 0.345 |
| AMD3100 | 0.383 |
| Plasinostat | 0.440 |
| Daunorubicin | 0.516 |
| ABT-199 | 0.551 |
| Idasanutlin | 0.719 |
| Cytarabine | 0.890 |

Result of proliferation test: Table 3 shows median values of Ci calculated with respect to the compound A and various anticancer drugs. The median values of Ci when the compound A and GSK-2879552, gilteritinib, ONO-4059 (tirabrutinib), azacytidine, midostaurin, or volasertib were used in combination were less than 1, revealing that the combined use of the compound A and these drugs showed the synergistic action. From the above, it was verified that the combined use of the compound A and these drugs exhibited a strong antitumor effect.

**[Table 3]**

| Drug to be combined | Ci median value in combined use with compound A |
|---|---|
| GSK-2879552 | 0.356 |
| Gilteritinib | 0.495 |
| ONO-4059 | 0.591 |
| Azacytidine | 0.666 |
| Midostaurin | 0.673 |
| Volasertib | 0.830 |

### Biological Example 2: In vitro test using human acute myeloid leukemia cell line for evaluating effect of combined use of compound A and ABBV-075

A human acute myeloid leukemia cell line CMK-11-5 was prepared in an RPMI medium containing 10% FBS (10% FBS-RPMI medium) at about 30000 cells/mL, and seeded in a 96 well plate at 50 µL/well. To this, 10% FBS-RPMI medium containing ABBV-075 having 4 times higher concentration than the final concentration was added at 25 µL/well. Furthermore, 10% FBS-RPMI medium containing the compound A having 4 times higher concentration than the final concentration was added at 25 µL/well (final volume: 100 µL/well, DMSO concentration: 0.2%). The plate was incubated at 37°C in humidified air including 5% CO₂ for 72 hours. After culturing, absorbance at 450 nm of a formazan product, proportional to the number of living cells, was measured using Cell Counting Kit-8. Percentage (%) of absorbance at 450 nm of each compound-treated group with respect to absorbance at 450 nm of the vehicle group was calculated.

The results are shown in Table 4. The absorbance at 450 nm when the compound A and ABBV-075 were used in combination was lowered as compared with that at the time of treatment by single use of each agent, showing that the combined use of the compound A and these drugs had a strong proliferation-suppressing action on the cell line. In addition, the median value of Ci in the combined use of the compound A and ABBV-075 was less than 1, revealing that the combined use of the compound A with ABBV-075 showed a synergistic action. From the above, it was verified that the combined use of the compound A and ABBV-075 exhibited a strong antitumor effect.

**[Table 4]**

| Drug to be combined | Ci median value in combined use with compound A |
|---|---|
| ABBV-075 | 0.533 |

### Biological Example 3: In vitro test using human acute lymphatic leukemia cell line for evaluating effect of combined use of compound A and various anticancer drugs

A human acute lymphatic leukemia cell line CCRF-HSB-2 was prepared in an IMDM medium containing 10% FBS (10% FBS-IMDM medium) at about 200000 cells/mL, and seeded in a 96 well plate at 50 µL/well. To this, 10% FBS-IMDM medium containing cytarabine, daunorubicin, or dexamethasone having 4 times higher concentration than the final concentration was added at 25 µL/well. Furthermore, 10% FBS-IMDM medium containing the compound A having 4 times higher concentration than the final concentration was added at 25 µL/well (final volume: 100 µL/well, DMSO concentration: 0.1%). The plate was incubated at 37°C in humidified air including 5% CO₂ for 72 hours. After culturing, relative luminescence unit (RLU), proportional to cellular endogenous ATP amount, was measured by Cell Titer-Glo Luminescent Cell Viability Assay (registered trademark). Percentage (%) of RLU of each compound-treated group relative to RLU of the vehicle group was measured.

The results are shown in Table 5 and FIGs. 1 to 3. The RLU when the compound A and dexamethasone, cytarabine, or daunorubicin were used in combination was lowered as compared with treatment with single use of each agent, showing that the combined use of the compound A and these drugs had a strong proliferation-suppressing action on the cell line. In addition, the median values of Ci in the combined use of the compound A and these drugs were all less than 1, revealing that the combined use of the compound A with these drugs had a synergistic action. From the above, it was verified that the combined use of the compound A and these drugs exhibited a strong antitumor effect.

**[Table 5]**

| Drug to be combined | Ci median value in combined use with compound A |
|---|---|
| Dexamethasone | 0.589 |
| Cytarabine | 0.696 |
| Daunorubicin | 0.936 |

### Biological Example 4: Evaluation of antitumor action by combined use of compound A and gemcitabine on mouse pancreatic cancer cell line PAN02 orthotopic transplantation model (in vivo)

### [Operation]

For the compound A, mouse feed CRF-1 containing 0.013% compound A was manufactured by Oriental Yeast Co., Ltd. so as to be administered in 20 mg/kg/day as administration via the diet. Gemcitabine was obtained from Wako Pure Chemical Industries, Ltd. C57/BL6 mouse pancreatic cancer cell line Pan02 was transplanted into the pancreas of the same type syngeneic mice (C57/BL6, female, 6-week old (Charles River Laboratories Japan, Inc.)) to produce Pan02 orthotopic implantation mice. On day 7 after transplantation, based on the body weight, the mice were assigned to four groups, i.e., a vehicle group, a gemcitabine group, compound A group, a combined use group of gemcitabine + compound A, and the compound A (each n = 8 to 9). The day on which group assignment was carried out was defined as Day 0. To the vehicle group and the compound A group, PBS (10 mL/kg, twice a week) was intraperitoneally administered. To the gemcitabine group and a combined use group of gemcitabine + compound A, gemcitabine (60 mg/10 mL/kg, twice a week) was administered by intraperitoneal administration. To the compound A group and the combined use group of gemcitabine + compound A, CRF-1 containing 0.013% compound A was fed from Day 0. From Day 0 to Day 95, the survival time of each mouse in each group was evaluated. Note here that from the viewpoint of the animal ethics, when mice had any one of conditions of not being able to take food and water because both hind limbs were paralyzed, showing no reaction to light stimulation given by a finger, having respiratory failure, crouching and not moving, showing loss of body weight by 20% for 2 to 3 days, and showing loss of body weight by 25% for 7 days, the mice were euthanatized, and the date was defined as dead date.

### [Results]

The results are shown in Table 6 and FIG. 4. A median value of the survival time (from the day of group assignment to the day of death) of the gemcitabine group was 67 days. The survival time was significantly extended as compared with 52 days in the vehicle group. A median value of the survival time of the combined use group of gemcitabine + compound A was 75 days, and the survival time was significantly extended as compared with the gemcitabine group.

From the above, it was verified that the combined use of the compound A and gemcitabine exhibited a strong antitumor effect.

**[Table 6]**

| Group name | Median value of survival days (day) | Number n (mice) |
|---|---|---|
| Vehicle | 52 | 9 |
| Gemcitabine | 67 | 8 |
| Compound A | 54 | 9 |
| Gemcitabine + compound A | 75 | 9 |

### Biological Example 5: Evaluation of antitumor action by combined use of compound A and gefitinib on gefitinib resistant human lung cancer cell line (in vitro)

### [Operation]

### (1) Cell culture

A human lung cancer cell line HCC827 having deletion mutation in EGFR gene exon 19, and having high sensitivity to an EGFR inhibitor was used. For, gefitinib-resistant HCC827 (HCC827GR (clone 2) and HCC827GR (clone 13)), which had been produced by long-term treatment of HCC827 with gefitinib in vitro at gradually increasing concentration, were used. Cells were subcultured in RPMI 1640 medium containing 10% inactivated fetal bovine serum and 2 mM L-glutamine under conditions of 5% CO₂ and 37°C. HCC827GR (clone 2) and HCC827GR (clone 13) were subcultured by adding gefitinib to be 42 µM in the above-mentioned medium.

### (2) Evaluation of cell survivability

Evaluation of the cell survivability was carried out using HCC827, HCC827GR (clone 2) and HCC827GR (clone 13) cells by the following procedure. Cells suspended in a RPMI1640 medium (growth medium) containing 10% inactivated fetal bovine serum and 2 mM L-glutamine were seeded in 384 well plate at a density of 2.5 × 10³ cells/25 µL per well, and cultured overnight in the conditions of 5% CO₂ 37°C. A growth medium containing (1) Gefitinib or a vehicle having two times higher concentration than the final concentration, and (2) the compound A or a vehicle having two times higher concentration than each final concentration were added each at 25 µL per well, and cultured for 72 hours at 5%CO₂ at 37°C. Note here that in the combined use group of the compound A and gefitinib, the compound A and gefitinib were prepared such that the molar concentration became 1:1. After 72 hours, cell survival rate was measured. Measurement was carried out using Celltiter-Blue (registered trademark) (manufactured by Promega Corporation) according to the procedure of the kit. In other words, Celltiter-Blue (registered trademark) reagent was added at 10 µL per well, and cultured for three hours under the conditions of 5% CO₂ and 37°C. After three hours, fluorescence intensity (excitation wavelength: 570 nm, fluorescence wavelength: 600 nm) was measured using Microplate Reader.

### (3) Data analysis

Analysis of the effect of combined use was carried out by calculating ED (effective dose)₅₀, ED₇₅, ED₉₀, and ED₉₅ using the fluorescence intensity, and calculating Combination index (CI) described in Advances in enzyme regulation, Vol. 22, 1984, pp. 27-55. CI is a generally used method for determining the intensity of the effect of the combined use. The score of CI was determined that CI<1 showed a synergistic effect, CI=1 showed additive effect, and CI>1 showed antagonism.

### [Results]

Table 7 shows evaluation results of the antitumor action by the combined use of the compound A and gefitinib using cell survivability of HCC827, HCC827GR (clone 2), and HCC827GR (clone 13) cells as an indicator. When the compound A and gefitinib were used in combination, in all cells, CI median values (median CI) were less than 1, showing a strong synergistic effect. From the above, it was verified that the combined use of the compound A and gefitinib exhibited a strong antitumor effect.

**[Table 7]**

| Cell | CI (ED50) | CI (ED75) | CI (ED90) | CI (ED95) | Median CI |
|---|---|---|---|---|---|
| HCC827 | 0.49 | 0.22 | 0.11 | 0.08 | 0.17 |
| HCC827GR (clone 2) | 0.03 | 0.02 | 0.02 | 0.03 | 0.03 |
| HCC827GR (clone 13) | 0.06 | 0.06 | 0.08 | 0.12 | 0.07 |

### Biological Example 6: Evaluation of antitumor action by combined use of compound A and vemurafenib, dabrafenib, or trametinib on vemurafenib-resistant human malignant melanoma cell line (in vitro)

### [Operation]

### (1) Cell culture

A vemurafenib-resistant cell line MEXF HT-144R Vemurafenib 6 µM and a vemurafenib-resistant cell line MEXF 394R Vemurafenib 4 µM were used. The vemurafenib-resistant cell line MEXF HT-144R Vemurafenib 6 µM was produced by long-term treatment of human malignant melanocytoma cell line MEXF HT-144 having activation mutation (V600E) in a BRAF gene and having high sensitivity to vemurafenib as a BRAF inhibitor with vemurafenib in vitro at gradually increasing concentration until final concentration of 6 µM. The vemurafenib-resistant cell line MEXF 394R Vemurafenib 4 µM was produced by long-term treatment of human malignant melanocytoma cell line MEXF 394 having activation mutation (V600K) in the BRAF gene and having high sensitivity to vemurafenib with vemurafenib in vitro at gradually increasing concentration until final concentration of 4 µM. Cells were subcultured in RPMI 1640 medium (containing L-glutamine and 25 mM HEPES) containing 10% inactivated fetal bovine serum and 0.1 mg/mL gentamicin under conditions of 5% CO₂ and 37°C by adding 4 µM of vemurafenib to the medium of MEXF 394R Vemurafenib 4 µM and 6 µM of vemurafenib to the medium of MEXF HT-144R Vemurafenib 6 µM, respectively.

### (2) Three-dimensional colony formation assay

Three-dimensional colony formation assay was carried out using MEXF 394R Vemurafenib 4 µM and MEXF HT-144R Vemurafenib 6 µM. The assay was carried out by the following procedure. Cells suspended in an IMDM medium containing 20% inactivated fetal bovine serum, 0.01% gentamicin, and 0.4% agar were seeded at a density of 2×10³ cells/50 µL to 1×10⁴ cells/50 µL per well. Furthermore, 100 µL of solution including a test substance or a vehicle was added thereto, and cultured for 24 hours under the conditions of 7.5% CO₂ and 37°C. On 24 hours after seeding, 90 µL of an IMDM medium containing 20% inactivated fetal bovine serum and 0.01% gentamicin (hereinafter, referred to as a "cell culture medium") was added to the agar layer. Then, the cell culture medium containing a test compound having 15 times higher concentration than the final concentration was added at 10 µL/well, and cultured for 8 to 13 days under conditions of 7.5% CO₂ and 37°C. After 8 to 13 days, the number of colonies were counted using Cellinsight NXT manufactured by Thermo Scientific. Note here that when a colony having a dimeter of more than 50 µm was formed, the formation of colony was determined.

### (3) Data analysis

Analysis of the effect of the combined use was carried out by calculating a CI value at each treatment concentration of each compound. From each CI value, a median value (Median CI) was calculated. It was determined that CI<1 showed a synergistic effect, CI=1 showed an additive effect, and CI>1 showed an antagonism.

### [Results]

Table 8 shows CI values in the combined use of the compound A and vemurafenib with respect to MEXF 394R Vemurafenib 4 µM. Table 9 shows CI values in the combined use of the compound A and vemurafenib with respect to MEXF HT-144R Vemurafenib 6 µM. Table 10 shows CI values in the combined use of the compound A and dabrafenib with respect to MEXF 394R Vemurafenib 4 µM. Table 11 shows CI value in the combined use of the compound A and trametinib with respect to MEXF 394R Vemurafenib 4 µM. CI value in the combined use of the compound A and trametinib with respect to MEXF HT-144R Vemurafenib 6 µM is shown in Table 12. In the case of the combined use of the compound A and any one of vemurafenib, dabrafenib, or trametinib, the Median CI is less than 1.0, showing the synergistic action. From the above, it was verified that the combined use of the compound A and vemurafenib, dabrafenib, or trametinib exhibited a strong antitumor effect. In addition, it is considered that the combined use of three agents, the compound A and vemurafenib or dabrafenib and trametinib may exhibit a strong antitumor action.

**[Table 8]**

| Antitumor effect by combined use of compound A and vemurafenib using MEXF 394R Vemurafenib 4 µM | | | |
|---|---|---|---|
| Vemurafenib µM | Compound A µM | CI | Median CI |
| 0.247 | 0.0123 | 0.729 | 0.752 |
| | 0.037 | 5.801 | |
| | 0.111 | 1.858 | |
| | 0.333 | 1.899 | |
| | 1 | 1.385 | |
| 0.741 | 0.0123 | 0.981 | |
| | 0.037 | 7.145 | |
| | 0.111 | 0.926 | |
| | 0.333 | 1.458 | |
| | 1 | 1.229 | |
| 2.22 | 0.0123 | 0.518 | |
| | 0.037 | 0.577 | |
| | 0.111 | 0.658 | |
| | 0.333 | 0.775 | |
| | 1 | 0.646 | |
| 6.67 | 0.0123 | 0.646 | |
| | 0.037 | 0.62 | |
| | 0.111 | 0.608 | |
| | 0.333 | 0.564 | |
| | 1 | 0.177 | |

**[Table 9]**

| Antitumor effect by combined use of compound A and vemurafenib using MEXF HT-144R Vemurafenib 6µM | | | |
|---|---|---|---|
| Vemurafenib µM | Compound A µM | CI | Median CI |
| 0.00914 | 0.0123 | 0.384 | 0.389 |
| | 0.037 | 0.578 | |
| | 0.111 | 0.269 | |
| | 0.333 | 0.226 | |
| | 1 | 0.189 | |
| 0.0274 | 0.0123 | 0.705 | |
| | 0.037 | 0.208 | |
| | 0.111 | 0.352 | |
| | 0.333 | 0.21 | |
| | 1 | 0.12 | |
| 0.0823 | 0.0123 | 0.47 | |
| | 0.037 | 0.409 | |
| | 0.111 | 0.354 | |
| | 0.333 | 0.363 | |
| | 1 | 0.285 | |
| 0.247 | 0.0123 | 0.708 | |
| | 0.037 | 0.635 | |
| | 0.111 | 0.516 | |
| | 0.333 | 0.488 | |
| | 1 | 0.389 | |
| 0.741 | 0.0123 | 0.94 | |
| | 0.037 | 0.773 | |
| | 0.111 | 0.67 | |
| | 0.333 | 0.5 | |
| | 1 | 0.361 | |

**[Table 10]**

| Antitumor effect by combined use of compound A and dabrafenib using MEXF394R Vemurafenib 4 µM | | | |
|---|---|---|---|
| Dabrafenib µM | Compound A µM | CI | Median CI |
| 0.0247 | 0.00411 | 3.053 | 0.972 |
| | 0.0123 | 7.871 | |
| | 0.037 | 22.402 | |
| | 0.111 | 65.936 | |
| | 0.333 | 19.293 | |
| 0.0741 | 0.00411 | 4.324 | |
| | 0.0123 | 9.142 | |
| | 0.037 | 1.211 | |
| | 0.111 | 1.39 | |
| | 0.333 | 1.835 | |
| 0.222 | 0.00411 | 0.596 | |
| | 0.0123 | 0.585 | |
| | 0.037 | 0.514 | |
| | 0.111 | 0.52 | |
| | 0.333 | 0.575 | |
| 0.667 | 0.00411 | 0.733 | |
| | 0.0123 | 0.676 | |
| | 0.037 | 0.638 | |
| | 0.111 | 0.644 | |
| | 0.333 | 0.562 | |

**[Table 11]**

| Antitumor effect by combined use of compound A and trametinib using MEXF 394R Vemurafenib 4 µM | | | |
|---|---|---|---|
| Trametinib µM | Compound A µM | CI | Median CI |
| 0.00037 | 0.0123 | 0.194 | 0.170 |
| | 0.037 | 0.201 | |
| | 0.111 | 0.154 | |
| | 0.333 | 0.133 | |
| | 1 | 0.078 | |
| 0.00111 | 0.0123 | 0.17 | |
| | 0.037 | 0.156 | |
| | 0.111 | 0.161 | |
| | 0.333 | 0.138 | |
| | 1 | 0.079 | |
| 0.00333 | 0.0123 | 0.238 | |
| | 0.037 | 0.238 | |
| | 0.111 | 0.168 | |
| | 0.333 | 0.168 | |
| | 1 | 0.099 | |
| 0.01 | 0.0123 | 0.504 | |
| | 0.037 | 0.297 | |
| | 0.111 | 0.297 | |
| | 0.333 | 0.201 | |
| | 1 | 0.056 | |
| 0.03 | 0.0123 | 0.892 | |
| | 0.037 | 0.892 | |
| | 0.111 | 0.892 | |
| | 0.333 | 0.603 | |
| | 1 | 0.167 | |

**[Table 12]**

| Antitumor effect by combined use of compound A and trametinib using MEXF HT-144R Vemurafenib 6 µM | | | |
|---|---|---|---|
| Trametinib µM | Compound A µM | CI | Median CI |
| 0.000123 | 0.0123 | 0.813 | 0.813 |
| | 0.037 | 183.48 | |
| | 0.111 | 2.262 | |
| | 0.333 | 1.924 | |
| | 1 | 0.477 | |
| 0.00037 | 0.0123 | 1.599 | |
| | 0.037 | 0.958 | |
| | 0.111 | 0.994 | |
| | 0.333 | 1.215 | |
| | 1 | 0.558 | |
| 0.00111 | 0.0123 | 0.939 | |
| | 0.037 | 0.553 | |
| | 0.111 | 0.51 | |
| | 0.333 | 0.46 | |
| | 1 | 0.462 | |
| 0.00333 | 0.0123 | 0.638 | |
| | 0.037 | 0.666 | |
| | 0.111 | 0.582 | |
| | 0.333 | 0.527 | |
| | 1 | 0.273 | |
| 0.01 | 0.0123 | 1.331 | |
| | 0.037 | 1.164 | |
| | 0.111 | 0.908 | |
| | 0.333 | 0.82 | |
| | 1 | 0.437 | |

### Biological Example 7: Evaluation of antitumor action by combined use of compound A and erlotinib or afatinib on EGFR (epidermal growth factor receptor) gene activating mutation positive human lung cancer cell line (in vitro)

### [Operation]

### (1) Cell culture

A human small cell lung cancer cell line NCI-H1650 in which EGFR was abnormally activated by deletion of exon 19 of EGFR gene (available from ATCC) and PC-3 (available from JCRB cell bank) were used. Cells were subcultured in a RPMI 1640 (hereinafter, referred to as a "medium") containing 10% inactivated fetal bovine serum and 2 mM L-glutamine under conditions of 5% CO₂ and 37°C.

### (2) Cell survivability evaluation

Cells suspended in a medium were seeded in a 96 well plate at a density of 1.0×10³ cells/100 µL/well overnight under conditions of 5% CO₂ and 37°C. The medium of each well was removed, and a medium containing (1) erlotinib, afatinib, or a vehicle having two times higher concentration than each final concentration, and (2) the compound A or a vehicle having two times higher concentration than each final concentration were added each at 50 µL per well, and cultured under conditions of 5% CO₂ and 37°C for 120 hours. After culturing, the number of survival cells was measured by quantifying the ATP of endogenous cells using CellTiter-Glo (registered trademark) Luminescent Cell Viability Assay (manufactured by Promega Corporation) according to the procedure of the kit.

### (3) Data analysis

The cell proliferation suppression rate (%) in each compound-treated group was calculated as a relative value with a vehicle treatment group defined to be 100%. Combination index (CI) value described in Advances in enzyme regulation, Vol. 22, 1984, pp. 27-55 using the calculated cell proliferation suppression rate (%). From each CI value, the median value (Median CI) was calculated, and it was determined that CI<1 showed a synergistic effect, CI=1 showed an additive effect, and CI>1 showed antagonism.

### [Results]

Table 13 shows evaluation results of the antitumor action by the combined use of the compound A and erlotinib using cell survivability of NCI-H1650 cells as an indicator; Table 14 shows evaluation results of the antitumor action by the combined use of the compound A and erlotinib using cell survivability of PC-3 cells as an indicator. Table 15 shows evaluation results of the antitumor action by the combined use of the compound A and afatinib using cell survivability of NCI-H1650 cells as an indicator; Table 16 shows evaluation results of the antitumor action by the combined use of the compound A and afatinib using cell survivability of PC-3 cells as an indicator. When the compound A and erlotinib or afatinib were used for treatment in combination, in any cells, the Median CI was less than 1, showing a synergistic effect. From the above, it was verified that the combined use of the compound A and erlotinib or afatinib exhibited a strong antitumor effect.

**[Table 13]**

| Antitumor effect by combined use of compound A and erlotinib using NCI-H1650 | | | |
|---|---|---|---|
| Erlotinib (µM) | Compound A (µM) | CI value | CI median value |
| 0.01 | 0.001 | 581.044 | 0.136 |
| 0.01 | 0.01 | 0.136 | |
| 0.01 | 0.1 | 0.411 | |
| 0.03 | 0.001 | 0.003 | |
| 0.03 | 0.01 | 0.028 | |
| 0.03 | 0.1 | 0.208 | |
| 0.1 | 0.001 | 0.017 | |
| 0.1 | 0.01 | 0.025 | |
| 0.1 | 0.1 | 0.207 | |
| 0.3 | 0.001 | 0.048 | |
| 0.3 | 0.01 | 0.026 | |
| 0.3 | 0.1 | 0.207 | |
| 1 | 0.001 | 0.317 | |
| 1 | 0.01 | 0.024 | |
| 1 | 0.1 | 0.19 | |

**[Table 14]**

| Antitumor effect by combined use of compound A and erlotinib using PC-3 | | | |
|---|---|---|---|
| Erlotinib (µM) | Compound A (µM) | CI value | CI median value |
| 0.01 | 0.001 | 1.779 | 0.501 |
| 0.01 | 0.01 | 1.089 | |
| 0.01 | 0.1 | 0.559 | |
| 0.03 | 0.001 | 0.501 | |
| 0.03 | 0.01 | 0.354 | |
| 0.03 | 0.1 | 0.189 | |
| 0.1 | 0.001 | 0.362 | |
| 0.1 | 0.01 | 0.356 | |
| 0.1 | 0.1 | 0.16 | |
| 0.3 | 0.001 | 0.827 | |
| 0.3 | 0.01 | 0.631 | |
| 0.3 | 0.1 | 0.177 | |
| 1 | 0.001 | 1.405 | |
| 1 | 0.01 | 1.59 | |
| 1 | 0.1 | 0.476 | |

**[Table 15]**

| Antitumor effect by combined use of compound A and afatinib using NCI-H1650 | | | |
|---|---|---|---|
| Afatinib (µM) | Compound A (µM) | CI value | CI median value |
| 0.01 | 0.001 | 0.597 | 0.099 |
| 0.01 | 0.01 | 0.098 | |
| 0.01 | 0.1 | 0.048 | |
| 0.03 | 0.001 | 0.722 | |
| 0.03 | 0.01 | 0.507 | |
| 0.03 | 0.1 | 0.046 | |
| 0.1 | 0.001 | 1.224 | |
| 0.1 | 0.01 | 0.385 | |
| 0.1 | 0.1 | 0.042 | |
| 0.3 | 0.001 | 3.208 | |
| 0.3 | 0.01 | 0.297 | |
| 0.3 | 0.1 | 0.068 | |
| 1 | 0.001 | 0.099 | |
| 1 | 0.01 | 0.04 | |
| 1 | 0.1 | 0.018 | |

**[Table 16]**

| Antitumor effect by combined use of compound A and afatinib using PC-3 | | | |
|---|---|---|---|
| Afatinib (µM) | Compound A (µM) | CI value | CI median value |
| 0.01 | 0.001 | 0.432 | 0.371 |
| 0.01 | 0.01 | 0.29 | |
| 0.01 | 0.1 | 0.074 | |
| 0.03 | 0.001 | 1.144 | |
| 0.03 | 0.01 | 0.617 | |
| 0.03 | 0.1 | 0.273 | |
| 0.1 | 0.001 | 2.014 | |
| 0.1 | 0.01 | 1.157 | |
| 0.1 | 0.1 | 0.371 | |
| 0.3 | 0.001 | 0.986 | |
| 0.3 | 0.01 | 1.35 | |
| 0.3 | 0.1 | 0.274 | |
| 1 | 0.001 | 0.183 | |
| 1 | 0.01 | 0.29 | |
| 1 | 0.1 | 0.1 | |

### Biological Example 8: Change of expression of Axl and Mer ligand in tumor after administration of anti-PD-1 antibody in mouse large-intestine cancer cell line MC38 subcutaneous cancer-bearing model (in vivo)

An anti-mouse PD-1 antibody 4H2, which had been prepared in KITAYAMA LABES CO., LTD., was used. C57/BL6 mouse large-intestine cancer cell line MC38 was subcutaneously transplanted into a dorsal part of the same type syngeneic mice (C57/BL6, female, 6-week old (Charles River Laboratories Japan, Inc.)) to produce MC38 subcutaneous cancer-bearing mice. On the day when the average of tumor volumes showed 150 mm³, group assignment was carried out based on the tumor volumes. The day of group assignment was started was defined as day 0. To the MC38 subcutaneous cancer-bearing mice, PBS (n=7) or 4H2 (20 mg/kg, n=7) was intraperitoneally administered on Day 0, and tumor was collected on Day 3. To the MC38 subcutaneous cancer-bearing mice, PBS (n=7) or 4H2 (20 mg/kg, n=7) was intraperitoneally administered on Day 0, and tumor was collected on Day 6. To the MC38 subcutaneous cancer-bearing mice, PBS (n=7) or 4H2 (20 mg/kg on Day 0, 10 mg/kg on Day 6, n=7) was intraperitoneally administered on Day 0 and Day 6, and collected on Day 10. The collected tumor was crushed in a frozen state, RNA was extracted using RNeasy Mini Kit (manufactured by Qiagen). Gene expression of Axl as a target of the compound A and Gas6 and Pros1 as a ligand of Mer was measured by RT-PCR. Results are shown as a ratio of a gene expression amount of HPRT that had been corrected as an internal standard relative to PBS-administered group. The results are shown in FIGs. 5 and 6.

Gas6 gene expression amount in the tumor was significantly higher (3.56 times) in the 4H2 administered group relative to the PBS administered group on Day 6. Gas6 gene expression amount in the tumor was significantly higher (1.58 times) in the 4H2 administered group relative to the Pros1 administered group on Day 10.

A plurality of research papers have reported the existence of negative feedback mechanism in which Gas6 and Pros1 are released from the activated immunocyte to activate Axl and Mer and negatively regulates the immune response (see, for example, Cell, Vol. 131, No. 6, pp. 1124-1136, 2007; Nature Reviews Cancer, Vol. 14, No. 12, pp. 769-785, 2014). From these reports and the results mentioned above, the compound A having an Axl inhibitory action is considered to show a strong tumor immune activation when the compound A is used in combination with the anti-PD-1 antibody.

### Biological Example 9: Evaluation of function of Axl and Mer in immune activation action by anti-CTLA-4 antibody using CTLA-4 Blockade Bioassay (in vitro)

CTLA-4 Blockade Bioassay kit and an anti-human CTLA-4 antibody were purchased from Promega Corporation. The effector cells included in this kit (CTLA-4 expression Jurkat cells) and antigen presenting cells (Raji cells) were diluted 5-fold and 10-fold in a medium. The both diluted cells in an equal amount were mixed with each other, PBS or anti-human CTLA-4 antibody (30 µg/mL) was treated (each n=3). After incubation for 16 hours, cells were collected, and RNA was extracted using RNeasy Mini Kit (manufactured by Qiagen). Thereafter, gene expression of Gas6 and Pros1 as ligand of Axl and Mer, as a target of the compound A, was measured by RT-PCR. Results are shown as a ratio of a gene expression amount of GAPDH as an internal standard relative to a PBS administered group. The results are shown in FIG. 7.

An expression amount of Gas6 was significantly increased by the anti-CTLA-4 antibody treatment (7.22 times). On the other hand, change of the expression of Pros1 was not observed. When documents described in Biological Example 1 and the results thereof are taken into account, the compound A having an Axl inhibitory action is considered to show a strong tumor immune activation when the compound A having an Axl inhibitory action is used in combination with the anti-CTLA-4 antibody.

### [Industrial Applicability]

Combination of the present invention exhibits a remarkable antitumor effect, and therefore is useful for cancer treatment.

## Claims

1. An agent for cancer treatment, comprising an Axl inhibitor as an active ingredient, which is administered in combination with an anticancer drug, wherein the Axl inhibitor is a compound represented by the general formula (I): 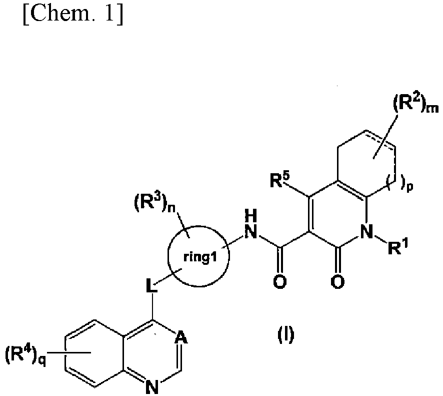 [wherein
R¹ represents (1) a C1-8 alkyl group optionally substituted with one to five R¹¹, (2) a C3-7 carbocyclic ring optionally substituted with one to five R¹², or (3) a 4- to 7-membered heterocycle optionally substituted with one to five R¹³, and herein when the C1-8 alkyl group represented by R¹ is a branched alkyl group, C1-3 alkyl groups branched from the same carbon atom, together with carbon atom bonded thereto, may form a saturated C3-7 carbocyclic ring;
R² represents (1) a C1-4 alkyl group, (2) a halogen atom, (3) a C1-4 haloalkyl group, (4) an oxo group, (5) an -OR²¹ group, or (6) an =NR²² group;
R³ represents (1) a C1-4 alkyl group, (2) a halogen atom, or (3) a C1-4 haloalkyl group;
R⁴ represents (1) a C1-4 alkoxy group, (2) a C1-4 haloalkyl group, or (3) an -OR⁴¹ group, (4) a C1-4 alkyl group, (5) a C2-4 alkenyloxy group, or (6) a C2-4 alkynyloxy group;
R⁵ represents (1) a hydrogen atom, (2) a C1-4 alkyl group, (3) a halogen atom, (4) a C1-4 haloalkyl group, or (5) an -OR²¹ group;
R¹¹ represents (1) an -OR¹⁰¹ group, (2) an SO₂R¹⁰² group, (3) an NR¹⁰³R¹⁰⁴ group, or (4) a C3-7 carbocyclic ring optionally substituted with one to three halogen atoms;
R¹² represents (1) a C1-8 alkyl group optionally substituted with a hydroxyl group, or (2) a halogen atom;
R¹³ represents (1) a C1-8 alkyl group optionally substituted with a hydroxyl group, or (2) a halogen atom;
R²¹ represents (1) a hydrogen atom, or (2) a C1-4 alkyl group;
R²² represents (1) a hydroxyl group, or (2) a C1-4 alkoxy group;
R⁴¹ represents (1) a hydrogen atom,
(2) a C1-8 alkyl group substituted with one or two substituents selected from the group consisting of (a) a 5- to 7-cyclic group optionally substituted with one to two substituents selected from the group consisting of (i) a C1-4 alkyl group, (ii) a C1-4 haloalkyl group, and (iii) a halogen atom, (b) NR⁴⁰¹R⁴⁰², (c) a hydroxyl group, and (d) a SO₂R⁴⁰³ group;
(3) a C2-8 alkenyl group substituted with one or two substituents selected from the group consisting of (a) a 5- to 7-cyclic group optionally substituted with one or two substituents selected from the group consisting of (i) a C1-4 alkyl group, (ii) a C1-4 haloalkyl group, and (iii) a halogen atom, (b) NR⁴⁰¹R⁴⁰², (c) a hydroxyl group, and (d) an SO₂R⁴⁰³ group; or
(4) a C2-8 alkynyl group substituted with one or two substituents selected from the group consisting of (a) a 5- to 7-cyclic group optionally substituted with one or two substituents selected from the group consisting of (i) a C1-4 alkyl group, (ii) a C1-4 haloalkyl group, and (iii) a halogen atom, (b) NR⁴⁰¹R⁴⁰², (c) a hydroxyl group, and (d) an SO₂R⁴⁰³ group,
R¹⁰¹ represents (1) a hydrogen atom, or (2) a C1-4 alkyl group;
R¹⁰² represents (1) a hydrogen atom, or (2) a C1-4 alkyl group;
R¹⁰³ and R¹⁰⁴ each independently represent (1) a hydrogen atom, or (2) a C1-4 alkyl group;
R⁴⁰¹ and R⁴⁰² each independently represent (1) a hydrogen atom, or (2) a C1-4 alkyl group;
R⁴⁰³ represents (1) a hydrogen atom, or (2) a C1-4 alkyl group;
A represents (1) CH, or (2) a nitrogen atom;
L represents (1) -O-, (2) -NH-, (3) -C(O)-, (4) -CR⁶R⁷ -, (5) -S-, (6) -S(O)-, or (7) -S(O)₂ -;
R⁶ and R⁷ each independently represent (1) a hydrogen atom, (2) a halogen atom, (3) a C1-4 alkyl group, (4) a hydroxyl group, or (5) NH₂;
ring1 represents a 5- to 7-membered cyclic group; 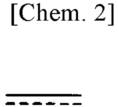
represents a single bond, or a double bond;
m represents an integer of 0 to 5;
n represents an integer of 0 to 5;
p represents an integer of 0 to 2;
q represents an integer of 0 to 4;
when m is 2 or more, a plurality of R^{2'}s may be the same as or different from each other, and herein when two R²'s represent a C1-3 alkyl group and are on the same carbon atom, the R²'s together with a carbon atom bonded thereto, may form a C3-7 saturated carbocyclic ring; when n is 2 or more, a plurality of R³'s may be the same as or different from each other; and when q is 2 or more, a plurality of R⁴'s may be the same as or different from each other], a salt thereof, a solvate thereof, an N-oxide thereof, or a prodrug thereof, wherein the anticancer drug does not include an immune checkpoint inhibitor other than an anti-CTLA-4 antibody.

2. The agent according to claim 1, wherein the Axl inhibitor is a compound represented by the general formula (I-1): 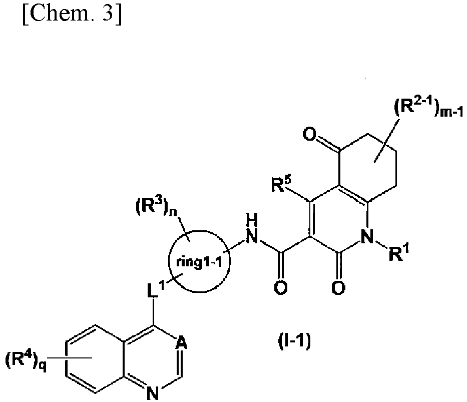
wherein R²⁻¹ represents (1) a C1-4 alkyl group, (2) a halogen atom, (3) a C1-4 haloalkyl group, (4) an -OR²¹ group, or (5) a =NR²² group;
m-1 represents an integer of 0 to 4;
L¹ represents (1) -O-, (2) -NH-, or (3) -C(O)-;
ring1-1 represents benzene or pyridine;
when m-1 is 2 or more, a plurality of R²⁻¹'s may be the same as or different from each other, and herein when the two R²⁻¹'s represent a C1-3 alkyl group and are on the same carbon atom, the R²⁻¹'s together with carbon atom bonded thereto, may form a C3-7 saturated carbocyclic ring; and
the other symbols have the same meanings as defined above], a salt thereof, a solvate thereof,
an N-oxide thereof, or a prodrug thereof.

3. The agent according to claim 1 or 2, wherein the Axl inhibitor is N-{5-[(6,7-dimethoxy-4-quinolinyl)oxy]-2-pyridinyl}-2,5-dioxo-1-phenyl-1,2,5,6,7,8-hexahydro-3-quinolinecarboxamide, a pharmaceutically acceptable salt thereof or a hydrate thereof.

4. The agent according to any one of claims 1 to 3, wherein the cancer is a blood cancer.

5. The agent according to claim 4, wherein the blood cancer is leukemia.

6. The agent according to claim 5, wherein the leukemia is acute myeloid leukemia or acute lymphoblastic leukemia.

7. The agent according to claim 4, wherein the anticancer drug is selected from the group consisting of a cytotoxic anticancer drug, a molecular target drug, a steroid preparation, a vitamin A derivative, an antiviral agent, L-asparaginase, arsenite, interferon α, cyclosporine, thalidomide, pomalidomide, and lenalidomide.

8. The agent according to claim 7, wherein the cytotoxic anticancer drug is selected from the group consisting of daunorubicin, azacytidine, and cytarabine.

9. The agent according to claim 7, wherein the molecular target drug is selected from the group consisting of AMD3100, ABBV-075, ABT-199, plasinostat, gilteritinib, midostaurin, GSK-2879552, idasanutlin, tirabrutinib, and volasertib.

10. The agent according to any one of claims 1 to 3, wherein the cancer is solid cancer.

11. The agent according to claim 10, wherein the solid cancer is head and neck cancer, nasopharyngeal cancer, esophageal cancer, gastro-esophageal junction cancer, esophageal adenocarcinoma, stomach cancer, large-intestine cancer, colon cancer, rectum cancer, small-intestine cancer, anal cancer, liver cancer, gallbladder cancer, bile duct cancer, biliary tract cancer, pancreatic cancer, thyroid cancer, parathyroid cancer, lung cancer, breast cancer, ovarian cancer, fallopian tube cancer, uterine cancer, vaginal cancer, vulvar cancer, penile cancer, kidney cancer, adrenal cancer, urothelial carcinoma, prostate cancer, testicular tumor, bone and soft tissue sarcoma, skin cancer, glioma, brain tumor, spine tumor, Kaposi's sarcoma, squamous cell carcinoma, pleural mesothelioma, primary peritoneal cancer, endocrine cancer, childhood cancer, or cancer of unknown primary.

12. The agent according to claim 10 or 11, wherein the solid cancer is pancreatic cancer, lung cancer, or skin cancer.

13. The agent according to claim 10, wherein the anticancer drug is selected from the group consisting of a cytotoxic anticancer drug, a molecular target drug, steroid, a hormone preparation, an aromatase inhibitor, and interferon α.

14. The agent according to claim 13, wherein the cytotoxic anticancer drug is an antimetabolite.

15. The agent according to claim 14, wherein the antimetabolite is gemcitabine.

16. The agent according to claim 13, wherein the molecular target drug is selected from the group consisting of an EGFR inhibitor, a BRAF inhibitor, and an MEK inhibitor.

17. The agent according to claim 16, wherein the EGFR inhibitor is gefitinib, erlotinib, or afatinib, the BRAF inhibitor is vemurafenib or dabrafenib, and the MEK inhibitor is trametinib.

18. The agent according to any one of claims 1 to 3, wherein the anticancer drug is an anti-CTLA-4 antibody.

19. The agent according to claim 18, wherein the anti-CTLA-4 antibody is selected from the group consisting of ipilimumab, tremelimumab, and AGEN-1884.

20. A method for treating cancer, the method including administering an effective amount of a compound represented by the general formula (I), a salt thereof, a solvate thereof, an N-oxide thereof, or a prodrug thereof according to claim 1 in combination with an anticancer drug to a patient in need of treatment of cancer, wherein the anticancer drug does not include an immune checkpoint inhibitor other than an anti-CTLA-4 antibody.

21. A compound represented by the general formula (I), a salt thereof, a solvate thereof, an N-oxide thereof, or a prodrug thereof according to claim 1, to be used in combination with an anticancer drug for cancer treatment, wherein the anticancer drug does not include an immune checkpoint inhibitor other than an anti-CTLA-4 antibody.

22. Use of a compound represented by the general formula (I), a salt thereof, a solvate thereof, an N-oxide thereof, or a prodrug thereof according to claim 1, for the manufacture of an agent for cancer treatment, wherein the agent for cancer treatment is administered in combination with an anticancer drug, wherein the anticancer drug does not include an immune checkpoint inhibitor other than an anti-CTLA-4 antibody.

23. An agent for cancer treatment, containing an anticancer drug as an active ingredient, which is administered in combination with an Axl inhibitor, wherein the Axl inhibitor is a compound represented by the general formula (I), a salt thereof, a solvate thereof, an N-oxide thereof, or a prodrug thereof according to claim 1, wherein the anticancer drug does not include an immune checkpoint inhibitor other than an anti-CTLA-4 antibody.

24. A pharmaceutical composition for cancer treatment, which is administered in combination with an Axl inhibitor and an anticancer drug, wherein the Axl inhibitor is a compound represented by the general formula (I), a salt thereof, a solvate thereof, an N-oxide thereof, or a prodrug thereof according to claim 1, wherein the anticancer drug does not include an immune checkpoint inhibitor other than an anti-CTLA-4 antibody.
